(19) Europäisches Patentamt / European Patent Office / Office européen des brevets

(11) **EP 2 398 454 B1**

(12) **FASCICULE DE BREVET EUROPEEN**

(45) Date de publication et mention
de la délivrance du brevet:
**07.01.2015 Bulletin 2015/02**

(21) Numéro de dépôt: **10708347.9**

(22) Date de dépôt: **15.02.2010**

(51) Int Cl.:
*A61K 8/60* *(2006.01)*      *B01F 17/56* *(2006.01)*
*B01F 17/38* *(2006.01)*      *A61K 8/06* *(2006.01)*

(86) Numéro de dépôt international:
**PCT/FR2010/050250**

(87) Numéro de publication internationale:
**WO 2010/094880 (26.08.2010 Gazette 2010/34)**

(54) **COMPOSITION EMULSIONNANTE PULVERULENTE D'ALKYL POLYGLYCOSIDES, UTILISATION POUR PREPARER DES EMULSIONS COSMETIQUES ET PROCEDE POUR LEUR PREPARATION**

PULVERFÖRMIGE EMULSIONSZUSAMMENSETZUNG AUS AKLYL-POLYGLYKOSIDEN, VERWENDUNG DAVON ZUR HERSTELLUNG VON KOSMETISCHEN EMULSIONEN UND HERSTELLUNGSVERFAHREN DAFÜR

POWDERY EMULSIFYING COMPOSITION OF ALKYL POLYGLYCOSIDES, USE THEREOF FOR PREPARING COSMETIC EMULSIONS, AND METHOD FOR PREPARING SAME

(84) Etats contractants désignés:
**AT BE BG CH CY CZ DE DK EE ES FI FR GB GR HR HU IE IS IT LI LT LU LV MC MK MT NL NO PL PT RO SE SI SK SM TR**

(30) Priorité: **19.02.2009 FR 0951074**

(43) Date de publication de la demande:
**28.12.2011 Bulletin 2011/52**

(73) Titulaire: **Société d'Exploitation de Produits pour les Industries Chimiques SEPPIC**
**75007 Paris (FR)**

(72) Inventeurs:
• **AMALRIC, Chantal**
**F-81700 Blan (FR)**
• **ROSO, Alicia**
**F-81710 Saix (FR)**
• **GORCE, Agnès**
**F-13009 Marseille (FR)**

(74) Mandataire: **Conan, Philippe Claude**
**L'Air Liquide SA**
**Direction de la Propriété Intellectuelle**
**75, quai d'Orsay**
**75321 Paris Cedex 07 (FR)**

(56) Documents cités:
**EP-A- 0 992 508      WO-A-96/37285**
**WO-A-97/18033      FR-A- 2 756 195**

• SNEZANA SAVIC ET AL: "Colloidal microstructure of binary systems and model creams stabilized with an alkylpolyglucoside non-ionic emulsifier" COLLOID AND POLYMER SCIENCE ; KOLLOID-ZEITSCHRIFT UND ZEITSCHRIFT FÜR POLYMERE, SPRINGER, BERLIN, DE, vol. 283, no. 4, 1 janvier 2005 (2005-01-01), pages 439-451, XP019340382 ISSN: 1435-1536

**Description**

L'invention concerne la fourniture de nouvelles compositions émulsionnantes utilisables dans la préparation de formulations cosmétiques et/ou pharmaceutiques notamment à usage topique.

Les agents émulsonnants dérivés de sucre ont été développés depuis plus d'une vingtaine d'année et leur succès commercial n'est plus à démontrer. Ils sont constitués d'un mélange d'alkyl polyglycosides et/ou d'alkényl polyglycosides et d'alcools gras, et se présentent sur le marché sous forme solide, qu'il s'agisse d'écailles, de poudre ou de perles. De tels agents émulsonnants sont décrits dans les demandes internationales publiées sous les numéros WO 92/06778, WO 95/13863, WO 96/37285, WO 98/47610 ou FR 2784904. Ils permettent de préparer des émulsions stables se présentant sous forme huile-dans-eau, eau-dans-huile. La présence d'une quantité d'alcools gras supérieure à 30% massique pour 100% de la masse de ces compositions induit une consistance et des propriétés sensorielles avantageuses, comme un toucher riche et onctueux de l'émulsion finale.

La demande de brevet FR 2 756 195 divulgue des compositions émulsionnantes destinées à la préparation d'émulsions à stabilité améliorée, et particulièrement destinées à préparer des émulsions fluides stables. Ces compositions comprennent :

i) de 30% à 90% en poids d'un mélange d'au moins un alkyl polyglycoside dont le radical alkyle linéaire ou ramifié, saturé ou insaturé comprend de 8 à 22 atomes de carbone et d'au moins un alkyl polyglycoside dont le radical alkyle est un radical oléyle ou un radical isostéaryle, et

ii) de 10% à 70% en poids d'au moins un alcool gras dont les radicaux alkyles, linéaires ou ramifiés, saturés ou insaturés, comprennent de 8 à 22 atomes de carbone.

La demande EP 0 992 508 divulgue des compositions émulsionnantes, destinées à améliorer la blancheur des émulsions préparées, et qui comprennent :

i) de 5% à 60% en poids d'un mélange d'alkyl polyglycosides comprenant pour 100% de sa masse :

◦ de 30% à 95% massique d'un mélange de cétyl polyglycosides et de stéary lpolyglycosides
◦ de 70% à 5% massique d'un mélange d'arachidyl polyglycosides et de béhényl polyglycosides ;

ii) de 95% à 40% en poids d'un ou plusieurs alcools gras dont les radicaux alkyles, linéaires ou ramifiés, saturés ou insaturés, comprennent de 14 à 22 atomes de carbone.

Les émulsions finales sont généralement préparées par dispersion de ces agents émulsionnants, soit dans l'eau ou dans une phase polaire, soit dans une phase huileuse. Ils sont donc préalablement portés à une température supérieure à leur point de fusion, qui selon les compositions commerciales est entre 60 et 80°C, avant d'être dispersés dans la phase aqueuse ou huileuse de l'émulsion finale, ce qui dans certains cas constitue un frein à leur expansion commerciale, car lesdites émulsions finales peuvent également comprendre des ingrédients thermosensibles et/ou volatils. De plus leur utilisation est également freinée pour fabriquer des émulsions huile-dans-eau car ils induisent parfois une inversion de phase qui se traduit par le changement non souhaité du sens de l'émulsion finale en émulsion eau-dans huile.

Pour résoudre les problèmes liés à une température trop élevée de préparation d'émulsions, on a cherché à développer des mélanges d'alkyl polyglycosides et d'alcools gras qui soient liquides à température ordinaire, pour éviter cette opération de fusion lors de la préparation de l'émulsion finale. Pour cela on a utilisé des alcools gras ramifiés ou insaturés pour conduire à de tels mélanges. De tels agents émulsionnants liquides sont décrits dans la demande internationale publiée sous le numéro WO 00/56438 ou encore dans la demande de brevet français publiée sous le numéro FR 2 830 464. De telles compositions liquides, à cause de leur caractère très lipophile, ne permettent pas la préparation d'émulsions huile-dans-eau suffisamment stables et/ou présentant des propriétés sensorielles suffisamment satisfaisantes pour le client final.

Dans le cadre de leurs recherches sur l'amélioration constante des agents émulsionnants pour préparer des émulsions "huile dans eau" qui soient dépourvus des inconvénients cités plus haut, les inventeurs ont mis au point de nouvelles compositions émulsionnantes, ainsi qu'un nouveau procédé de préparation d'émulsions huile-dans-eau, permettant de préparer des émulsions stables en présence d'ingrédients thermosensibles et/ou volatils, sans apparition de phénomènes d'inversion de phase lors de leur préparation et procurant aux dites émulsions des propriétés sensorielles avantageuses, comme un toucher riche et onctueux.

Selon un premier aspect, l'invention a pour objet une composition pulvérulente C1 comprenant pour 100% de sa masse :

- de 5% massique à 70% massique, plus particulièrement de 10% massique à 50 % massique d'au moins un composé de formule (I) :

$$R\text{-}O\text{-}(G)_x\text{-}H \qquad (I)$$

dans laquelle le radical R représente un radical aliphatique linéaire et saturé comportant de 12 à 22 atomes de carbone, G représente le reste d'un sucre réducteur choisi parmi le groupe constitué du glucose, du xylose et de l'arabinose, et x représente un nombre décimal supérieur ou égal à 1 et inférieur ou égal à 10 ;

- de 95% massique à 30% massique, plus particulièrement de 90% massique à 50 % massique d'un ou plusieurs alcools de formule (II) :

$$R'\text{-}OH \qquad (II)$$

dans laquelle le radical R', identique ou différent du radical R tel que défini ci-dessus, représente un radical aliphatique linéaire saturé comportant de 12 à 22 atomes de carbone, et dans laquelle au moins 90% en volume de particules sont de diamètre inférieur ou égal à 250 micromètres, et plus particulièrement de diamètre inférieur ou égal à 150 micromètres.

Par radical aliphatique linéaire saturé comportant de 12 à 22 atomes de carbone, on désigne notamment pour R dans la formule (I) telle que définie ci-dessus, par exemple les radicaux dodécyle, tétradécyle, hexadécyle, octadécyle, eicosyle et docosyle.

Par radical aliphatique linéaire saturé comportant de 12 à 22 atomes de carbone, on désigne notamment pour R' dans la formule (II) telle que définie ci-dessus, un radical identique ou différent du radical R de la formule (I) telle que définie ci-dessus, par exemple les radicaux dodécyle, tétradécyle, hexadécyle, octadécyle, eicosyle et docosyle.

Dans la formule (I) telle que définie précédemment, x est un nombre décimal qui représente le degré moyen de polymérisation du reste G.

Lorsque x est un nombre entier, $(G)_x$ est le reste polymérique de rang x du reste G.

Lorsque x est un nombre décimal, la formule (I) représente un mélange de composés :

$a_1\, R\text{-}O\text{-}(G)_1\text{-}H + a_2\, R\text{-}O\text{-}(G)_2\text{-}H + a_3\, R\text{-}O\text{-}(G)_3\text{-}H + ... + a_q\, R\text{-}O\text{-}(G)_q\text{-}H$ avec q représentant un nombre entier compris entre 1 et 10 et dans les proportions molaires $a_1, a_2, a_3,... a_q$ telles que :

$$\sum_{q=10}^{q=1} a_q = 1 \; ; \; a_1 > 0$$

Selon un autre aspect particulier de la présente invention, dans la formule (I) telle que définie précédemment, x est compris entre 1,05 et 5, et plus particulièrement entre 1,05 et 2.

Par reste d'un sucre réducteur, on désigne pour G dans la formule (I) telle que définie précédemment, un reste de dérivés saccharidiques qui ne présentent pas dans leurs structures de liaison glycosidique établie entre un carbone anomérique et l'oxygène d'un groupement acétal tels qu'ils sont définis dans l'ouvrage de référence : « Biochemistry », Daniel Voet/Judith G. Voet, p. 250, John Wyley & Sons, 1990. La structure oligomérique $(G)_x$ peut se présenter sous toute forme d'isomérie, qu'il s'agisse d'isomérie optique, d'isomérie géométrique ou d'isomérie de position ; elle peut aussi représenter des mélanges d'isomères.

Dans la formule (I) telle que définie ci-dessus, le radical R est lié à G par le carbone anomérique du reste saccharide, de manière à former une fonction acétal.

Selon un aspect particulier de la présente invention, par reste d'un sucre réducteur, on désigne dans la formule (I) telle que définie précédemment, le reste du glucose.

Selon un autre aspect particulier de la présente invention, par reste d'un sucre réducteur, on désigne dans la formule (I) telle que définie précédemment, le reste du du xylose.

Par l'expression : "la composition pulvérulente C1 comprend au moins 90% en volume de particules de diamètre inférieur ou égal à 250 micromètres, et plus particulièrement de diamètre inférieur ou égal à 150 micromètres", on signifie dans le cadre de la présente invention, que la composition pulvérulente $C_1$ est une poudre de particules assimilées à des sphères dont 90% en volume d'entre elles ont un diamètre inférieur ou égal à 250 micromètres, et plus particulièrement inférieur ou égal à 150 micromètres.

La détermination de ce paramètre est réalisée au moyen d'un analyseur à diffraction laser, par exemple le "granulomètre laser MALVERN Mastersize™ 2000, équipé d'un dispositif de dispersion, par exemple le dispositif de dispersion de type MS1-Small Volume Sample Dispersion™, et relié à un logiciel de calcul, qui permet d'obtenir un dififractogramme consistant en une superposition des images de diffraction de chaque taille de particules représentées dans la poudre analysée. Dans l'analyse des données ainsi recueillies, une distribution de taille initiale est estimée et le diffractogramme théorique est calculé, puis comparé avec les données réelles enregistrées. Les différences entre les données estimées et les données réelles sont ensuite minimisées en utilisant la méthode des moindres carrées. Le logiciel calcule ensuite la distribution en volume en tant que résultat fondamental et toute autre information est déduite de ce résultat en supposant que les particules ont une forme sphérique.

Cette méthode de détermination est particulièrement bien adaptée à la caractérisation des poudres dont les particules qui la constituent sont assimilées à des sphères de diamètres compris entre 3000 micromètres et 0,1 micromètres, et pour des poudres sèches. L'utilisation de ce type de méthode a particulièrement montré de bons résultats pour des tailles de particules supérieures à 10 micromètres [P. Bowen, "Particle Size Distribution Measurement from Millimeters to Nanometers and from Rods to Platelets"; J. Dispersion Science and Technology, 23(5), pp. 631-662 (2002)].

La préparation de la composition pulvérulente C1 objet de la présente invention, comprend :

- une étape A) de glycosylation des alcools de formule (II) telle que définie précédemment par un sucre réducteur, tel que défini précédemment, suivie
- d'une étape B) de broyage des produits obtenus sous la forme de solides à l'issue de l'étape A).

La réaction de glycosylation, mise en oeuvre dans l'étape A) des alcools de formule (II) telle que définie précédemment par un sucre réducteur est bien connue de l'homme du métier et est décrite dans les demandes de brevet publiées sous les numéros documents WO92/06778, WO96/37286, WO95/13863, WO98/47610 et FR2784904. Elle est généralement mise en oeuvre dans un réacteur en présence d'un système catalytique acide, en maîtrisant le rapport stoechiométrique entre les deux réactants, et avec agitation mécanique dans des conditions de température et de vide partiel prédéterminées, par exemple à une température comprise entre 70°C et 130°C et sous un vide partiel compris entre 300 mbar (3.10$^4$ Pa) et 20 mbar (2.10$^3$ Pa).

Par système catalytique acide, on désigne les acides forts comme l'acide sulfurique, l'acide chlorhydrique, l'acide phosphorique, l'acide nitrique, l'acide hypophosphoreux, l'acide méthanesulfonique, l'acide (para-toluène) sulfonique, l'acide (trifluorométhane) sulfonique, ou les résines échangeuses d'ions. Un tel procédé de glycosylation peut être complété, si nécessaire ou si désiré, par des opérations de neutralisation, de filtration et de décoloration.

Le broyage de la composition solide obtenue à l'issue de l'étape A) décrite ci-dessus et se présentant sous la forme d'un solide non divisé comme par exemple sous la forme de perles ou d'écailles, est effectué lors de l'étape B) au moyen d'un broyeur à couteaux ou au moyen d'un dispositif utilisant la technique de micronisation par cryobroyage, de façon à obtenir à l'issue de cette étape B) de broyage une composition pulvérulente C1 objet de la présente invention.

La technique de micronisation par cryobroyage, consistant à congeler la composition solide issue de l'étape A) décrite ci-dessus au moyen d'azote liquide ou de dioxyde de carbone liquide avant la réalisation de l'opération de broyage, est préférentiellement choisie pour préparer la composition émulsionnante pulvérulente C1 objet de la présente invention.

Selon un aspect plus particulier, la composition pulvérulente C1 objet de la présente invention comprend une proportion massique non nulle d'au moins un composé de formule (Ia), correspondant à la formule (I) telle que définie précédemment dans laquelle R représente un radical aliphatique linéaire saturé comportant de 20 à 22 atomes de carbone, et une proportion massique non nulle d'au moins un alcool de formule (IIa), correspondant à la formule (II) telle que définie précédemment dans laquelle R' représente un radical aliphatique linéaire saturé comportant de 20 à 22 atomes de carbone.

Selon un autre aspect plus particulier, la composition pulvérulente C1 objet de la présente invention comprend, pour 100% de sa masse :

- de 5% massique à 20% massique d'au moins un composé de formule (Ia) ;
- de 1,5% massique à 10% massique d'au moins un composé de formule (Ib) correspondant à la formule (I) telle que définie précédemment, dans laquelle R représente un radical aliphatique linéaire saturé comportant de 12 à 14 atomes de carbone ;
- de 1% massique à 10% massique d'au moins un composé de formule (Ic) correspondant à la formule (I) telle que définie précédemment, dans laquelle R représente un radical aliphatique linéaire saturé comportant de 16 à 18 atomes de carbone ;
- de 45% massique à 80% massique d'au moins un composé de formule (IIa) ;
- de 5% massique à 10% massique d'au moins un composé de formule (IIb) correspondant à la formule (II) telle que définie précédemment dans laquelle R' représente un radical aliphatique linéaire saturé comportant de 12 à 14 atomes de carbone ; et

- de 0% massique à 10% massique d'au moins un composé de formule (IIc) correspondant à la formule (II) telle que définie précédemment dans laquelle R' représente un radical aliphatique linéaire saturé comportant de 16 à 18 atomes de carbone.

Selon un autre aspect plus particulier, la composition pulvérulente C1 objet de la présente invention comprend pour 100% de sa masse :

- de 5% massique à 70% massique d'au moins un composé de formule (Ic), et
- de 95% massique à 30% massique d'au moins un composé de formule (IIc).

Selon un autre aspect plus particulier, la composition pulvérulente C1 objet de la présente invention, comprend au moins 90% en volume de particules de diamètre inférieur ou égal à 100 micromètres, et plus particulièrement de diamètre inférieur ou égal à 50 micromètres.

Selon un autre aspect, l'invention a pour objet l'utilisation d'une composition pulvérulente C1 telle que décrite précédemment, comme agent émulsionnant pour préparer des émulsions. Celles-ci peuvent être de type huile-dans-eau, de type eau-dans-huile, de type eau-dans-huile-dans-eau et de type huile-dans-eau-dans-huile, et lesdites émulsions peuvent se présenter sous la forme de crèmes, de laits, de gels crèmes, d'émulsions fluides, d'émulsions fluides vaporisables.

Dans le cadre de l'invention, on entend par « émulsion fluide » une émulsion dont l'écoulement au travers d'une coupe d'écoulement ISO 2431 de 6 millimètres commence moins de 5 secondes après l'enlèvement de l'obturateur (test selon la norme internationale ISO 2431). A titre d'émulsions fluides, on peut notamment citer les laits, en particulier les laits du type huile-dans-eau, à usage cosmétique ou hygiénique comme les laits démaquillants, les laits corporels, les laits solaires ou les laits autobronzants. A titre d'émulsions fluides vaporisables, on peut notamment citer les émulsions fluides pulvérisables comprenant un gaz propulseur cosmétiquement acceptable.

Selon un autre aspect, l'invention a pour objet un procédé de préparation d'une émulsion cosmétique huile-dans-eau, par émulsification d'une phase grasse P1 avec une phase aqueuse P2, comprenant au moins une étape a) de préparation de ladite phase grasse P1 comprenant le mélange d'au moins une ou de plusieurs huiles et/ou d'une ou de plusieurs cires, avec une quantité efficace de la composition pulvérulente C1, telle que définie précédemment.

De manière surprenante, du fait que au moins 90% en volume de particules de la composition pulvérulente C1 sont de diamètre inférieur ou égal à 250 micromètres, plus particulièrement de diamètre inférieur ou égal à 150 micromètres, plus particulièrement de diamètre inférieur ou égal à 100 micromètre et encore plus particulièrement inférieur ou égal à 50 micromètres.

Dans le procédé objet de l'invention, l'étape a) de préparation de la phase grasse P1 par mélange d'une ou plusieurs huiles et/ou d'une ou plusieurs cires avec une composition pulvérulente C1 telle que décrite précédemment, peut être avantageusement mise en oeuvre à une température inférieure au point de fusion théorique de la composition pulvérulente C1, à une température inférieure ou égale à 70°C, plus particulièrement à une température comprise entre 20°C et 60°C, et encore plus particulièrement à une température comprise entre 20°C et 40°C.

L'étape a) du procédé objet de l'invention peut être réalisée par le moyen de tout dispositif de mélange connu de l'homme du métier, comme par exemple par le moyen d'un dispositif d'agitation équipé d'un mobile de type « ancre », à des vitesses d'agitations comprises entre 80 tours/minute et 1000 tours/minute, et comme par exemple par le moyen d'un dispositif d'agitation de type rotor-stator à des vitesses d'agitation comprises entre 1000 tours/minute et 8000 tours /minute.

Dans le procédé tel que défini ci-dessus, par quantité efficace de composition pulvérulente C1, on entend une proportion massique généralement comprise entre 1% massique et 10% massique de la masse totale de l'émulsion.

Dans le procédé tel que défini ci-dessus, parmi les huiles constitutives de la phase grasse P1 préparée lors de l'étape a), on peut citer :

- les huiles volatiles. Par « huile volatile », on entend dans la présente description toute huile susceptibble de s'évaporer au contact de la peau et/ou de faible point éclair, c'est-à-dire ayant un point éclair inférieur à environ 100°C, et plus particulièrement un point éclair compris entre 30°C et 85°C. Comme huile volatile, on peut citer les huiles hydrocarbonées à chaîne ramifiée, comportant de préférence de 6 à 20 atomes de carbone comme par exemple les isoparaffines, l'isohexadécane, identifié dans Chemical Abstracts par le numéro RN = 93685-80-4 et qui est un mélange d'isoparaffines en $C_{12}$, $C_{16}$ et $C_{20}$ contenant au moins 97% d'isoparaffines en $C_{16}$, parmi lesquelles le constituant principal est le 2,2,4,6,8,8-heptaméthyl nonane (RN = 4390-04-9), et l'isododécane, et les huiles de silicones volatiles, linéaires ou cycliques, telles que les cyclométhicones comme la cyclohexadiméthylsiloxane, la cyclopentadiméthylsiloxane.
- les huiles minérales telles que l'huile de paraffine, l'huile de vaseline, les isoparaffines ou les huiles blanches minérales ;

- les huiles d'origine animale, telles que le squalène ou le squalane ;
- les huiles végétales, telles que le phytosqualane, l'huile d'amandes douces, l'huile de coprah, l'huile de ricin, l'huile de jojoba, l'huile d'olive, l'huile de colza, l'huile d'arachide, l'huile de tournesol, l'huile de germes de blé, l'huile de germes de maïs, l'huile de soja, l'huile de coton, l'huile de luzerne, l'huile de pavot, l'huile de potiron, l'huile d'onagre, l'huile de millet, l'huile d'orge, l'huile de seigle, l'huile de carthame, l'huile de bancoulier, l'huile de passiflore, l'huile de noisette, l'huile de palme, le beurre de karité, l'huile de noyau d'abricot, l'huile de calophyllum, l'huile de sysymbrium, l'huile d'avocat, l'huile de calendula, les huiles issues de fleurs ou de légumes ;
- les huiles végétales éthoxylées ;
- les huiles synthétiques comme les esters d'acides gras tels que le myristate de butyle, le myristate de propyle, le myristate de cétyle, le palmitate d'isopropyle, le stéarate de butyle, le stéarate d'hexadécyle, le stéarate d'isopropyle, le stéarate d'octyle, le stéarate d'isocétyle, l'oléate dodécyle, le laurate d'hexyle, le dicaprylate de propylèneglycol, les esters dérivés d'acide lanolique, tels que le lanolate d'isopropyle, le lanolate d'isocétyle, les monoglycérides, diglycérides et triglycérides d'acides gras comme le triheptanoate de glycérol, les alkylbenzoates, les huiles hydrogénées, les polyalphaoléfines, les polyoléfines comme le polyisobutène, les isoalcanes de synthèse, le polydécène hydrogéné ou le polyisobutène hydrogéné, commercialisé en France par la société Ets B. Rossow et Cie sous le nom PARLEAM - POLYSYNLANE™, cité dans : Michel and Irene Ash ; Thesaurus of Chemical Products, Chemical Publishing Co, Inc. 1986 Volume I, page 211 (ISBN 0 7131 3603 0), les huiles perfluorées ; et
- les huiles de silicone comme les diméthylpolysiloxanes, les méthylphényl - polysiloxanes, les silicones modifiées par des amines, les silicones modifiés par des acides gras, les silicones modifiés par des alcools, les silicones modifiés par des alcools et des acides gras, des silicones modifiés par des groupements polyéther, des silicones époxy modifiés, des silicones modifiées par des groupements fluorés, des silicones cycliques et des silicones modifiées par des groupements alkyles.

Dans le procédé tel que défini ci-dessus, parmi les cires constitutives de la phase grasse P1 préparée lors de l'étape a), on peut citer la cire d'abeille, la cire de carnauba, la cire de candelilla, la cire d'ouricoury, la cire du Japon, la cire de fibre de liège, la cire de canne à sucre, les cires de paraffines, les cires de lignite, les cires microcristallines, la cire de lanoline ; l'ozokérite ; la cire de polyéthylène, les cires de silicone ; les cires végétales ; les alcools gras et les acides gras solides à température ambiante ; les glycérides solides à température ambiante.

Dans le procédé objet de l'invention et tel que décrit ci-dessus, la phase grasse P1 préparée lors de l'étape a) peut comprendre en outre des actifs lipophiles et/ou lipodispersibles, et plus particulièrement des actifs lipophiles et/ou lipodispersibles sensibles à la température. Parmi les actifs lipophiles et/ou lipodispersibles pouvant être compris dans la phase grasse P1 préparée lors de l'étape a) du procédé objet de l'invention, on peut citer les composés liposolubles et/ou lipodispersibles ayant une action éclaircissante ou dépigmentante, une action hydratante, une action tenseur, une action apaisante ou relaxante, une action anti inflammatoire, une action amincissante, une action lipolytique, une action drainante, une action détoxifiante, une action une action énergisante, décontractante, une action stimulante, une action émolliente, une action neuromodulatrice, une action protectrice, une action purifiante, séborégulatrice, antichute, une action anti-âge, une action raffermissante, restructurante, antiradicalaire, ou antioxydante. De tels principes actifs sont par exemple les protéines N-acylées, les peptides N-acylés comme par exemple le MATRIXIL™, les acides aminés N - acylés se présentant sous leurs formes acides comme par exemple le N-(ω-undécylènoyl) phénylalanine commercialisé par la société SEPPIC sous l'appellation SEPIWHITE™MSH, le N-octanoyl glycine commercialisé par la société SEPPIC sous l'appellation LIPACIDE™C8G, le N-undécylènoyl glycine commercialisé par la société SEPPIC sous l'appellation LIPACIDE™UG, les hydrolysats partiels de protéines N-acylés, les acides aminés, les peptides, les hydrolysats totaux de protéines, les vitamines liposolubles, les dérivés de vitamines liposolubles comme par exemple le Rétinol, la vitamine E et ses dérivés, les lipides en général, les lipides tels que les céramides ou les phospholipides, les actifs ayant une action amincissante ou lipolytique comme la caféine ou ses dérivés, le panthénol et ses dérivés comme le SEPICAP™ MP, les actifs anti-âge comme le SEPILIFT™ DPHP, le LIPACIDE™ PVB, les actifs augmentant la synthèse des composants de la matrice extracellulaire par exemple, le collagène, les élastines, les glycosaminoglycanes, les actifs agissant favorablement sur la communication cellulaire chimique comme les cytokines ou physiques comme les intégrines, les actifs créant une sensation de « chauffe » sur la peau comme les activateurs de la microcirculation cutanée (exemple des nicotinates) ou des produits créant une sensation de « fraîcheur » sur la peau (exemple du menthol et de ses dérivés). Parmi les actifs liposolubles et/ou lipodispersibles sensibles à la chaleur, on peut citer par exemple la vitamine E, les dérivés de la vitamine E ; les huiles essentielles reconnues pour leurs actions antiseptiques comme par exemple le Thymus Vulgaris, pour leurs actions anti inflammatoires comme par exemple le Syzygium Aromaticum, pour leurs actions cicatrisante et protectrices comme par exemple la Lavandulla Officinalis ; les parfums ; les bases odorantes et olfactives ; le carotène ; la vitamine A et ses dérivés ; les acides gras polyinsaturés ; les colorants.

Selon un aspect particulier, le procédé de préparation d'une émulsion huile-dans-eau objet de l'invention décrit précédemment comprend en outre :

- au moins une <u>étape b)</u> de mélange de la phase grasse P1 obtenue à l'issue de <u>l'étape a)</u>, avec une quantité efficace d'un polyélectrolyte pour obtenir une phase P'1, puis
- au moins une <u>étape c)</u> d'émulsification de ladite phase P'1 obtenue à l'issue de <u>l'étape b)</u> avec ladite aqueuse P2.

Par « polymère polyélectrolyte » on entend dans la présente description des polymères comprenant dans leur squelette au moins un monomère comportant au moins une charge électrique ionique associée à un contre-ion. Parmi les polymères de type polyélectrolyte susceptibles d'être mis en oeuvre dans <u>l'étape b)</u> du procédé objet de l'invention, on peut citer :

- des homopolymères à base d'un monomère possédant une fonction acide fort, partiellement ou totalement salifiée,
- des homopolymères à base d'un monomère possédant une fonction acide faible, partiellement ou totalement salifiée,
- des homopolymères à base d'un monomère cationique,
- des polymères à base d'au moins un monomère possédant une fonction acide fort, partiellement ou totalement salifiée, polymérisé :

    o soit avec au moins un monomère possédant une fonction acide faible, partiellement ou totalement salifiée,
    o soit avec au moins un monomère neutre,

- des polymères à base d'au moins un monomère cationique polymérisé avec au moins un monomère neutre,
- des polymères à base d'au moins un monomère possédant une fonction acide faible, partiellement ou totalement salifiée, polymérisé :

    o soit avec au moins un monomère possédant une fonction acide faible, partiellement ou totalement salifiée,
    o soit avec au moins un monomère neutre.

- des polymères à base d'au moins un monomère possédant une chaine alkyle hydrophobe polymérisé avec au moins un monomère possédant une fonction acide faible, partiellement ou totalement salifiée, et/ou avec au moins un monomère possédant une fonction acide fort, partiellement ou totalement salifiée, et/ou avec un monomère neutre.

Dans ce contexte , l'expression « partiellement ou totalement salifiée » signifie que les fonctions acide fort ou acide faible sont partiellement ou totalement salifiées sous forme notamment de sels de métal alcalin, tel que le sel de sodium ou le sel de potassium, de sel d'ammonium ou de sel d'amino-alcool, tel que par exemple le sel de monoéthanolamine.

La fonction acide fort du monomère peut notamment être la fonction acide sulfonique ou la fonction acide phosphonique, lesdites fonctions étant partiellement ou totalement salifiées. Ledit monomère sera avantageusement choisi parmi l'acide styrène sulfonique ou le méthacrylate de 2-sulfo éthyle, l'acide styrène phosphonique, partiellement ou totalement salifié, l'acide-2-méthyl-(1-oxo-2-propényl)amino]-1-propane sulfonique (AMPS) partiellement ou totalement salifié sous forme de sel de sodium, de sel de potassium, d'ammonium ou de sel de monoéthanolamine.

La fonction acide faible du monomère peut notamment être la fonction acide carboxylique, partiellement ou totalement salifiée. Ledit monomère peut notamment être choisi parmi l'acide acrylique, l'acide méthacrylique, l'acide itaconique ou l'acide maléique partiellement ou totalement salifié sous forme de sel de sodium, de sel de potassium, de sel d'ammonium ou de sel de monoéthanolamine.

Lorsque le polymère est un copolymère à base d'un monomère possédant une fonction acide fort partiellement ou totalement salifiée, copolymérisé avec au moins un monomère neutre, ledit monomère neutre est choisi parmi l'acrylamide, le méthacrylamide, la vinylpyrrolidone, le N,N-diméthyl acrylamide, l'acrylate de 2-hydroxy éthyle, le méthacrylate de 2-hydroxy éthyle, le méthacrylate de 2,3-dihydroxy propyle ou un dérivé éthoxylé de poids moléculaire compris entre 400 et 1000 de chacun de ces esters hydroxylés, le tris(hydroxyméthyl)-acrylamido-méthane ou le tris(hydroxyméthyl)-méthacrylamido-méthane ou un dérivé éthoxylé de poids moléculaire compris entre 400 et 1500 de ces amides.

Les polymères polyélectrolytes décrits ci-dessus peuvent être « ramifiés » ou « réticulés ». Par « polymère ramifié » on désigne un polymère non linéaire qui possède des chaînes pendantes de manière à obtenir, lorsque ce polymère est mis en solution dans l'eau, un fort état d'enchevêtrement conduisant à des viscosités à bas gradients très importantes. Par « polymère réticulé » on désigne un polymère non linéaire se présentant à l'état d'un réseau tridimensionnel insoluble dans l'eau, mais gonflable à l'eau et conduisant donc à l'obtention d'un gel chimique.

Lorsque le polymère est réticulé et/ou ramifié, l'agent de réticulation et/ou l'agent de ramification est notamment choisi parmi les composés diéthyléniques, les composés polyéthyléniques, et tout particulièrement parmi l'acide diallyloxyacétique ou un de sels et notamment son sel de sodium, la triallylamine, le triméthylol propanetriacrylate, le diméthacrylate d'éthylène glycol, le diacrylate de diéthylène glycol, la diallylurée ou le méthylène (bis)acrylamide.

L'agent de réticulation et/ou de ramification est généralement utilisé dans une proportion molaire exprimée par rapport aux monomères mis en oeuvre, de 0,005% molaire à 1% molaire, de préférence de 0,01% molaire à 0,2%molaire, et encore plus préférentiellement de 0,01% molaire à 0,1% molaire.

Parmi les polymères de type polyélectrolytes qui conviennent tout particulièrement pour la réalisation de l'étape b) du procédé objet de l'invention, on peut citer les copolymères de l'acide acrylique partiellement ou totalement salifié et de l'acide-2-méthyl-(1-oxo-2-propényl)amino]-1-propane sulfonique (AMPS) partiellement ou totalement salifié, les copolymères de l'acrylamide et de l'acide-2-méthyl-(1-oxo-2-propényl)amino]-1-propane sulfonique (AMPS) partiellement ou totalement salifié, les copolymères de l'acrylate de 2-hydroxyéthyle et de l'acide-2-méthyl-(1-oxo-2-propényl)amino]-1-propane sulfonique (AMPS) partiellement ou totalement salifié, l'homopolymère de l'acide acrylique partiellement ou totalement salifié, l'homopolymère de l'acide-2-méthyl-(1-oxo-2-propényl)amino]-1-propane sulfonique (AMPS) partiellement ou totalement salifié, les copolymères du chlorure d'acryloyléthyl triméthyl ammonium et de l'acrylamide, les copolymères de l'AMPS et de la vinylpyrrolidone, les copolymère de l'acide acrylique et d'acrylates d'alkyle dont la chaîne carbonée comprend entre dix et trente atomes de carbone.

De tels polymères sont obtenus par les procédés de préparation connus de l'homme du métier parmi lesquels on peut citer les procédés de polymérisation par suspension, les procédés de polymérisation par voie précipitante en présence d'un solvant, les procédés de polymérisation par suspension inverse, les procédés de polymérisation en phase inverse.

De tels polymères polyélectrolytes se présentent sous la forme de poudres, de suspensions, d'émulsions ou d'émulsions inverses.

De tels polymères polyélectrolytes sont commercialisés sous les appellations SIMULGEL™ EG, SEPIGEL™ 305, SIMULGEL™ NS, SIMULGEL™ 800, SIMULGEL™ A, SIMULGEL™ EPG, SIMULGEL™ INS, SIMULGEL™ FL, SIMULGEL™ SMS 88, SEPIGEL™ 501, SEPIGEL™ 502, SEPIPLUS™ 250, SEPIPLUS™ 265, SEPIPLUS™ 400, SEPIPLUS™ S, SEPINOV™ EMT 10, CARBOPOL™, ULTREZ™ 10, ACULYN™, PEMULEN™ TR1, PEMULEN™ TR2, LUVIGEL™ EM, SALCARE™ SC91, SALCARE™ SC92, SALCARE™ SC95, SALCARE™ SC96, FLOCARE™ ET100, FLOCARE™ ET58, HISPAGEL™, NOVEMER™ EC1, ARISTOFLEX™ AVC, ARISTOFLEX™ HBM, RAPITHIX™ A60, RAPITHIX™ A100, COSMEDIA™ SP et STABILEZE™ 06.

Par quantité efficace de polymère polyélectrolyte, on signifie que La masse sèche dudit polymère polyélectrolyte constitue entre 0,1% massique et 4% massique, de préférence entre 0,5% massique et 2% massique pour 100% de la masse de la phase grasse P'1 préparée dans l'étape b) du procédé objet de l'invention si un tel polymère polyélectrolyte résulte d'un procédé de polymérisation par voie précipitante.

La masse sèche dudit polymère polyélectrolyte constitue entre 0,25% massique et 4% massique, de préférence entre 0,5% massique et 2% massique pour 100% de la masse de la phase grasse P'1 préparée dans l'étape b) du procédé objet de l'invention, si un tel polymère polyélectrolyte résulte d'un procédé de polymérisation en émulsion inverse.

L'expression "cosmétiquement acceptable " utilisée dans la définition de la phase aqueuse mise en oeuvre lors de l'étape c) du procédé de préparation d'une émulsion cosmétique huile-dans-eau objet de l'invention, qualifie, selon la directive du Conseil de la Communauté Economique Européenne N°76/768/CEE du 27 juillet 1976 modifiée par la directive N°93/35/CEE du 14 juin 1993, toute substance ou préparation destinée à être mise en contact avec les diverses parties du corps humain (épiderme, système pileux et capillaire, ongles, lèvres et organes génitaux) ou avec les dents et les muqueuses buccales en vue, exclusivement et principalement, de les nettoyer, de les parfumer, d'en modifier l'aspect et/ou d'en corriger les odeurs corporelles et/ou de les protéger ou de les maintenir en bon état. Une phase aqueuse cosmétiquement acceptable contient classiquement de l'eau, un ou plusieurs solvants organique cosmétiquement acceptables, un mélange d'eau et d'un ou plusieurs solvants organiques. Les solvants cosmétiquement acceptables peuvent plus particulièrement être choisis parmi les alcools polyhydriques comme par exemple le glycérol, le diglycérol, les oligomères du glycérol, l'éthylène glycol, le propylène glycol, le butylène glycol, l'hexylène glycol, le diéthylène glycol, le xylitol, l'érythritol, le sorbitol, ou les alcools hydrosolubles tels que l'éthanol, l'isopropanol ou le butanol.

Dans le procédé objet de l'invention et tel que décrit ci-dessus, la phase aqueuse cosmétiquement acceptable P2 peut comprendre en outre des actifs hydrosolubles et/ou hydrodispersibles, et plus particulièrement des actifs hydrosolubles et/ou hydrodispersibles sensibles à la température. Parmi les actifs hydrosolubles et/ou hydrodispersibles pouvant être compris dans la phase aqueuse cosmétiquement acceptable P2 , on peut citer les composés hydrosolubles et/ou hydrodispersibles ayant une action éclaircissante ou dépigmentante, une action hydratante, une action tenseur, une action apaisante ou relaxante, une action anti inflammatoire, une action amincissante, une action lipolytique, une action drainante, une action détoxifiante, une action une action énergisante, décontractante, une action stimulante, une action émoliente, une action neuromodulatrice, une action protectrice, une action purifiante, séborégulatrice, antichute, une action anti-age, une action raffermissante, restructurante, antiradicalaire, ou antioxydante. De tels principes actifs sont par exemple les vitamines hydrosolubles et/ou hydrodispersibles comme par exemple la vitamine C et ses dérivés, le magnésium ascorbyl phosphate et ses dérivés, l'ascorbyl glucoside, l'acide phytique, les acides de fruits, les extraits aqueux ou hydroalcooliques ou hydroglycoliques de polyphénols, de quinoa, de panais, de potentille, de raisin, de vin, les extraits d'olives, les extraits de marc, les polyols (par exemple, la glycérine ou le butylène glycol), les dérivés de lait, ou les différents composants entrant dans la composition du NMF (natural moisturizing factor) par exemples l'urée, l'acide pyrrolidone carboxylique ou les dérivés de cet acide, les acides aminés, les sels minéraux, les sucres ou les dérivés des sucres, les polysaccharides ou leurs dérivés, les hydroxyacides par exemple l'acide lactique, les extraits végétaux aqueux ou hydroalcooliques ou hydroglycoliques, tels que les extraits végétaux riches en tanins, les extraits végétaux riches en isoflavones ou les extraits végétaux riches en terpènes, les extraits d'algues d'eau douce ou marines, les extraits marins en général comme les coraux, les extraits bactériens ; les minéraux comme les GIVOBIO™, les dérivés de calcium, de magnésium, de cuivre, de cobalt, de zinc, de lithium, ou de manganèse, les sels d'argent ou d'or ; les actifs ayant une propriété énergisante ou stimulante comme le SEPITONIC™ M3 ou le Physiogényl™ ; les actifs auto-bronzants comme par exemple la dihydroxyacétone et l'érythrulose ; les actifs hydratant ou restructurant de l'épiderme comme par exemple l'AQUAXYL™, les acides aminés N-acylés se présentant sous leurs formes salifiées comme par exemple les sels de sodium, ou de potassium, ou d'ammonium, ou d'amino-alcools du N-(ω-undécylènoyl) phénylalanine, de l'octanoylglycine , de l'undécylénoylglycine, les sels d'hydrolysâts partiels de protéines N-acylés, les acides aminés, les hydrolysâts totaux de protéines. Parmi les actifs hydrosolubles et/ou hydrodispersibles sensibles à la chaleur, on peut citer par exemple les actifs auto-bronzants comme par exemple la dihydroxyacétone et l'érythrulose ; le miel ; les eaux florales comme par exemple l'eau de jeunes pousses d'orge ; la vitamine C et ses dérivés ; l'eau oxygénée.

De manière surprenante, du fait que au moins 90% en volume de particules de la composition émulsionnante pulvérulente C1 sont de diamètre inférieur ou égal à 250 micromètres, plus particulièrement de diamètre inférieur ou égal à 150 micromètres, plus particulièrement de diamètre inférieur ou égal à 100 micromètre et encore plus particulièrement inférieur ou égal à 50 micromètres,

Dans le procédé objet de l'invention, l'étape c) de mélange de la phase P'1 obtenue à l'issue de l'étape b) avec la phase aqueuse P2 cosmétiquement acceptable peut être avantageusement mise en oeuvre à une température inférieure ou égale à 70°C, plus particulièrement à une température comprise entre 20°C et 60°C, et encore plus particulièrement à une température comprise entre 20°C et 40°C.

L'étape c) du procédé objet de l'invention peut être réalisée par le moyen de tout dispositif de mélange connu de l'homme du métier, comme par exemple par le moyen d'un dispositif d'agitation équipé d'un mobile de type « ancre », à des vitesses d'agitations comprises entre 80 tours/minute et 1000 tours/minute, et comme par exemple par le moyen d'un dispositif d'agitation de type rotor-stator à des vitesses d'agitation comprises entre 1000 tours/minute et 8000 tours /minute.

Selon un aspect particulier de l'invention, une variante du procédé de préparation d'une émulsion cosmétique huile-dans-eau tel que défini précédemment, comprend au moins une étape b', d'émulsification de ladite phase grasse P1 obtenue à l'étape a) avec ledit polymère polyélectrolyte et ladite phase aqueuse P2.

De manière surprenante, du fait que au moins 90% en volume de particules de la composition émulsionnante pulvérulente C1 sont de diamètre inférieur ou égal à 250 micromètres, plus particulièrement de diamètre inférieur ou égal à 150 micromètres, plus particulièrement de diamètre inférieur ou égal à 100 micromètre et encore plus particulièrement inférieur ou égal à 50 micromètres, dans la variante du procédé objet de l'invention, l'étape b') de mélange de la phase grasse P1 obtenue à l'issue de l'étape a) avec un polymère polyélectrolyte tel que décrit précédemment et avec une phase aqueuse P2 telle que décrite précédemment, peut être avantageusement mise en oeuvre à une température inférieure à 70°C, plus particulièrement à une température comprise entre 20°C et 60°C, et encore plus particulièrement à une température comprise entre 20°C et 40°C.

L'étape b') de la variante du procédé objet de l'invention peut être réalisée par le moyen de tout dispositif de mélange connu de l'homme du métier, comme par exemple par le moyen d'un dispositif d'agitation équipé d'un mobile de type « ancre », à des vitesses d'agitations comprises entre 80 tours/minute et 1000 tours/minute, comme par exemple par le moyen d'un dispositif d'agitation de type rotor-stator à des vitesses d'agitation comprises entre 1000 tours/minute et 8000 tours /minute.

Selon un autre aspect, l'invention a pour objet un procédé de préparation d'une émulsion cosmétique huile-dans-eau par émulsification d'une phase grasse avec une phase aqueuse D1 avec une phase grasse D2 comprenant une étape a1) de préparation de ladite phase aqueuse D1 comprenant le mélange d'une quantité efficace de la composition émulsionnante pulvérulente C1, telle que définie à l'une des revendications 1 à 5, avec de l'eau ou un dispersion aqueuse d'un ou de plusieurs ingrédients hydrophiles cosmétiquement acceptables.

Par ingrédients hydrophiles cosmétiquement acceptables, on entend par exemple les actifs hydrosolubles et/ou hydrodispersibles tels que décrits précédemment, et plus particulièrement des actifs hydrosolubles et/ou hydrodispersibles sensibles à la température tels que décrits précédemment.

L'étape a1) du procédé objet de l'invention peut être réalisée par le moyen de tout dispositif de mélange connu de l'homme du métier, comme par exemple par le moyen d'un dispositif d'agitation équipé d'un mobile de type « ancre », à des vitesses d'agitations comprises entre 80 tours/minute et 1000 tours/minute, et comme par exemple par le moyen d'un dispositif d'agitation de type rotor-stator à des vitesses d'agitation comprises entre 1000 tours/minute et 8000 tours /minute.

De manière surprenante, du fait que au moins 90% en volume de particules de la composition émulsionnante pulvérulente C1 sont de diamètre inférieur ou égal à 250 micromètres, plus particulièrement de diamètre inférieur ou égal à 150 micromètres, plus particulièrement de diamètre inférieur ou égal à 100 micromètre et encore plus particulièrement inférieur ou égal à 50 micromètres,

Dans le procédé objet de l'invention, l'étape a1) de préparation de la dispersion D1 par mélange de la composition émulsionnante pulvérulente C1 telle que définie précédemment avec la phase aqueuse cosmétiquement acceptable, peut être avantageusement mise en oeuvre à une température inférieure ou égale à 70°C, plus particulièrement à une température comprise entre 20°C et 60°C, et encore plus particulièrement à une température comprise entre 20°C et 40°C.

Selon un aspect particulier, le procédé de préparation d'une émulsion huile-dans-eau objet de l'invention décrit précédemment comprend en outre

- une étape b1) de préparation de ladite phase grasse D2 comprenant le mélange d'au moins une ou de plusieurs huiles et/ou d'une ou de plusieurs cires, avec une quantité efficace d'un un polymère polyélectrolyte ;

- une étape c1) de mélange de ladite phase grasse D2 obtenue à l'issue de l'étape b1) avec ladite phase aqueuse D1 obtenue à l'issue de l'étape a1).

La phase grasse D2 peut comprendre en outre des actifs liposolubles et/ou lipodispersibles tels que décrits précédemment, et plus particulièrement des actifs liposolubles et/ou lipodispersibles sensibles à la température tels que décrits précédemment.

De manière surprenante, du fait que au moins 90% en volume de particules de la composition émulsionnante pulvérulente C1 sont de diamètre inférieur ou égal à 250 micromètres, plus particulièrement de diamètre inférieur ou égal à 150 micromètres, plus particulièrement de diamètre inférieur ou égal à 100 micromètre et encore plus particulièrement inférieur ou égal à 50 micromètres, dans le procédé objet de l'invention, l'étape c1) de préparation de la dispersion D1 par mélange de la composition émulsionnante pulvérulente C1 telle que définie précédemment avec la phase aqueuse cosmétiquement acceptable, peut être avantageusement mise en oeuvre à une température inférieure ou égale à 70°C, plus particulièrement à une température comprise entre 20°C et 60°C, et encore plus particulièrement à une température comprise entre 20°C et 40°C.

L'étape c1) du procédé objet de l'invention peut être réalisée par le moyen de tout dispositif de mélange connu de l'homme du métier, comme par exemple par le moyen d'un dispositif d'agitation équipé d'un mobile de type « ancre », à des vitesses d'agitations comprises entre 80 tours/minute et 1000 tours/minute, comme par exemple par le moyen d'un dispositif d'agitation de type rotor-stator à des vitesses d'agitation comprises entre 1000 tours/minute et 8000 tours /minute.

Selon un autre aspect, l'invention a pour objet une préparation à usage topique comprenant une émulsion huile-dans-eau préparée selon les procédés et leurs variantes décrits ci-dessus.

L'expression "à usage topique" utilisée dans la définition de la préparation objet de l'invention, signifie que ladite préparation est mise en oeuvre par application sur la peau, les cheveux, le cuir chevelu ou les muqueuses, qu'il s'agisse d'une application directe dans le cas d'une préparation cosmétique, dermocosmétique, dermo-pharmaceutique ou pharmaceutique ou d'une application indirecte par exemple dans le cas d'un produit de soin corporel sous forme de lingette en textile ou en papier ou de produits sanitaires destinés à être en contact. avec la peau ou les muqueuses.

Les préparations à usage topique, comprenant une émulsion huile-dans-eau préparée selon les procédés et leurs variantes décrits précédemment, et objets de la présente invention peuvent se présenter sous la forme de crèmes, de laits, de gels crèmes, de lotions fluides, de lotions fluides vaporisables.

De façon générale ces préparations à usage topique, comprenant une émulsion huile-dans-eau préparée selon les procédés et leurs variantes décrits précédemment, et objet de la présente invention, comportent également des excipients et ou des principes actifs habituellement mis en oeuvre dans le domaine des formulations à usage topique, en particulier cosmétiques, dermocosmétiques, pharmaceutiques ou dermo-pharmaceutiques, comme les tensioactifs épaississants et/ou gélifiants, les stabilisants, les composés filmogènes, les agents hydrotropes, les agents plastifiants, les agents émulsionnants et co-émulsionnants, les agents opacifiants, les agents nacrants, les agents surgraissants, les séquestrants, les agents chélatants, les antioxydants, les parfums, les conservateurs, les agents conditionneurs, les agents blanchissants destinés à la décoloration des poils et de la peau, les principes actifs destinés à apporter une action traitante vis à vis de la peau ou des cheveux, les filtres solaires, les charges minérales ou les pigments, les particules procurant un effet visuel ou destinées à l'encapsulation d'actifs, les particules exfoliantes, les agents de texture.

Comme exemples de tensioactifs épaississants et/ou gélifiants éventuellement présents dans la préparation à usage topique, comprenant une émulsion huile-dans-eau préparée selon les procédés et leurs variantes décrits précédemment, et objet de la présente invention, on peut citer :

- les esters gras d'alkylpolyglycosides éventuellement alkoxylés, et tout particulièrement les esters de méthylpolyglucoside éthoxylés tels que le PEG 120 méthyl glucose trioléate et le PEG 120 méthyl glucose dioléate commercialisés respectivement sous les appellations GLUCAMATE™ LT et GLUMATE™ DOE120

- Les esters gras alkoxylés tels que le PEG 150 pentaérythrytyl tétrastéarate commercialisé sous l'appellation CROTHIX™ DS53, le PEG 55 propylene glycol oléate commercialisé sous l'appellation ANTIL™ 141.

- les carbamates de polyalkylène glycols à chaînes grasses tels que le PPG 14 laureth isophoryl dicarbamate commercialisé sous l'appellation ELFACOS™ T211, le PPG 14 palmeth 60 hexyl dicarbamate commercialisé sous l'appellation ELFACOS™ GT2125.

Comme exemples d'agents opacifiants et/ou nacrants éventuellement présents dans la préparation à usage topique, comprenant une émulsion huile-dans-eau préparée selon les procédés et leurs variantes décrits précédemment, et objet de la présente invention, on peut citer les palmitates ou les stéarates ou les hydroxystéarates de sodium ou de magnésium, les monostéarates ou distéarates d'éthylène ou de polyéthylène glycol, les alcools gras, les homopolymères et copolymères de styrène tels que le styrène acrylate copolymère commercialisé sous l'appellation MONTOPOL™ OP1 par la société SEPPIC.

Comme exemples d'agents de texture éventuellement présents dans la préparation à usage topique, comprenant une émulsion huile-dans-eau préparée selon les procédés et leurs variantes décrits précédemment, et objet de la présente invention, on peut citer des dérivés N-acylés d'acides aminés, comme la lauroyl lysine commercialisée sous l'appellation AMINOHOPE™LL par la société AJINOMOTO, les polyméthacrylates de méthyle commercialisés sous l'appellation MICROPEARL™ par la société SEPPIC, le nylon-12.

Comme exemples de filtres solaires éventuellement présents dans la préparation à usage topique, comprenant une émulsion huile-dans-eau préparée selon les procédés et leurs variantes décrits précédemment, et objet de la présente invention, on peut citer tous ceux figurant dans la directive cosmétique 76/768/CEE modifiée annexe VII.

Les exemples suivants illustrent l'invention, sans toutefois la limiter.

**Exemple 1 : préparation de compositions émulsionnante pulvérulentes objets de l'invention.**

**Exemple 1.1** : **préparation d'une composition émulsionnante pulvérulente (X) constituée d'alcool arachidylique, d'alcool béhénique, d'arachidyl polyglucosides et de béhényl polyglucoside.**

On introduit 950,0 g d'un mélange d'alcool arachidylique et d'alcool béhénique, dans un ratio massique alcool arachidylique/alcool béhénique 70/30, dans un réacteur en verre à double enveloppe, dans laquelle circule un fluide caloporteur, et muni d'une agitation efficace, à une température de 80°C pour permettre la fonte totale du mélange d'alcools. 97,5 g de glucose anhydre sont alors ajoutés progressivement au milieu réactionnel pour permettre sa dispersion homogène. Le mélange homogène est maintenu à une température de 80°C pendant 30 minutes, puis 1,43 g d'acide sulfurique à 98% et 0,95 g d'acide hypophosphoreux à 50% sont introduits dans la dispersion homogène préalablement préparée. Le milieu réactionnel est placé sous un vide partiel de 90 mbars à 45 mbars, et maintenu à une température de 100°C-105°C pendant une durée de 5 heures avec évacuation de l'eau formée au moyen d'un montage de distillation. Le milieu réactionnel est ensuite refroidi à 85°C-90°C et neutralisé par ajout de 1,3 g de soude à 40 %, pour amener le pH d'une solution à 5 % de ce mélange à une valeur d'environ 6,5. La composition ainsi obtenue est ensuite vidangée à une température de 70°C et maintenue à température ambiante de façon à obtenir un solide homogène non divisé (composition $X_1$).

La composition ($X_1$) obtenue à l'étape précédente sous la forme d'un solide homogène non divisée est ensuite broyée par le moyen d'un dispositif de cryobroyage de marque Micronis, équipé d'un refroidissement à l'azote liquide et d'un broyeur à broche bi rotor fonctionnant à une fréquence de 60 Hz, de façon à obtenir une composition (X) se présentant sous la forme d'une poudre fine dont les caractéristiques analytiques sont rassemblées dans le tableau 1 ci-dessous.

**Exemple 1.2** : **préparation d'une composition émulsionnante pulvérulente (Y) constituée d'alcool cétylique, d'alcool stéarylique, de cetyl polyglucosides et de stéaryl polyglucoside.**

On introduit 952,0 g d'un mélange d'alcool cétylique et d'alcool stéarylique, dans un ratio massique alcool cétylique/alcool stéarylique 50/50, dans un réacteur en verre à double enveloppe, dans laquelle circule un fluide caloporteur, et muni d'une agitation efficace, à une température de 80°C pour permettre la fonte totale du mélange d'alcools. 125,0 g de glucose anhydre sont alors ajoutés progressivement au milieu réactionnel pour permettre sa dispersion homogène. Le mélange homogène est maintenu à une température de 80°C pendant 30 minutes, puis 1,12 g d'acide sulfurique à 98% et 0,86 g d'acide hypophosphoreux à 50% sont introduits dans la dispersion homogène préalablement préparée. Le milieu réactionnel est placé sous un vide partiel de 90 mbars à 45 mbars, et maintenu à une température de 100°C-105°C pendant une durée de 5 heures avec évacuation de l'eau formée au moyen d'un montage de distillation.

Le milieu réactionnel est ensuite refroidi à 85°C-90°C et neutralisé par ajout de 1,3 g de soude à 40 %, pour amener le pH d'une solution à 5 % de ce mélange à une valeur d'environ 6,5. La composition ainsi obtenue est ensuite vidangée à une température de 70°C et maintenue à température ambiante de façon à obtenir un solide homogène (composition $Y_1$).

La composition ($Y_1$) obtenue à l'étape précédente sous la forme d'un solide homogène est ensuite broyée par le moyen d'un dispositif de cryobroyage de marque Micronis , équipé d'un refroidissement à l'azote liquide et d'un broyeur à broche bi rotor fonctionnant à une fréquence de 60 Hz, de façon à obtenir une composition (Y) se présentant sous la forme d'une poudre fine dont les caractéristiques analytiques sont rassemblées dans le tableau 1 ci-dessous.

**Exemple 1.3 : préparation d'une composition émulsionnante pulvérulente ($Y_2$) constituée d'alcool cétylique, d'alcool stéarylique, de cetyl polyglucosides et de stéaryl polyglucoside.**

On introduit 952,0 g d'un mélange d'alcool cétylique et d'alcool stéarylique, dans un ratio massique alcool cétylique/alcool stéarylique 50/50, dans un réacteur en verre à double enveloppe, dans laquelle circule un fluide caloporteur, et muni d'une agitation efficace, à une température de 80°C pour permettre la fonte totale du mélange d'alcools. 125,0 g de glucose anhydre sont alors ajoutés progressivement au milieu réactionnel pour permettre sa dispersion homogène. Le mélange homogène est maintenu à une température de 80°C pendant 30 minutes, puis 1,12 g d'acide sulfurique à 98% et 0,86 g d'acide hypophosphoreux à 50% sont introduits dans la dispersion homogène préalablement préparée. Le milieu réactionnel est placé sous un vide partiel de 90 mbars à 45 mbars, et maintenu à une température de 100°C-105°C pendant une durée de 5 heures avec évacuation de l'eau formée au moyen d'un montage de distillation. Le milieu réactionnel est ensuite refroidi à 85°C-90°C et neutralisé par ajout de 1,3 g de soude à 40 %, pour amener le pH d'une solution à 5 % de ce mélange à une valeur d'environ 6,5. Le milieu réactionnel ainsi neutralisé est alors introduit dans un évaporateur à film à couche mince, sous une pression réduite de 3 à 5 mbars et avec une température de paroi de 210°C, de façon à distiller une partie des alcools cétyliques et stéaryliques résiduels. Le culot de distillation est alors vidangé et refroidi à la température ambiante de façon à obtenir une composition ($Y'_2$) se présentant sous la forme d'un solide homogène.

La composition ($Y'_2$) obtenue à l'étape précédente sous la forme d'un solide homogène est ensuite broyée par le moyen d'un dispositif de cryobroyage de marque Micronis , équipé d'un refroidissement à l'azote liquide et d'un broyeur à broche bi rotor fonctionnant à une fréquence de 60 Hz, de façon à obtenir une composition ($Y_2$) se présentant sous la forme d'une poudre fine dont les caractéristiques analytiques sont rassemblées dans le tableau 1 ci-dessous.

**Exemple 1.4 : préparation d'une composition émulsionnante pulvérulente (W) constituée d'alcool laurique, d'alcool myristique, cétylique, d'alcool stéarylique, d'alcool arachidylique, d'alcool béhénique, de lauryl polyglucosides, de myristyl polyglucosides, de cetyl polyglucosides, de stéaryl polyglucoside, d'arachidyl polyglucoside et de béhényl polyglucosides.**

On introduit 955,0 g d'un mélange d'alcools constitué en pourcentage massique de 16,5% d'alcool laurique, de 18,5% d'alcool myristique, de 4,5% d'un mélange d'alcool cétylique et d'alcool stéarylique dans un ratio massique alcool cétylique/alcool stéarylique de 50/50, 60,5% d'un mélange d'alcool arachidylique et d'alcool béhénique, dans un ratio massique alcool arachidylique/alcool béhénique 70/30, dans un réacteur en verre à double enveloppe, dans laquelle circule un fluide caloporteur, et muni d'une agitation efficace, à une température de 80°C pour permettre la fonte totale du mélange d'alcools. On ajoute progressivement sur ce mélange d'alcools la quantité de glucose nécessaire de sorte que le rapport molaire entre le mélange d'alcools gras et le glucose soit de 6/1.

Le mélange homogène est maintenu à une température de 80°C pendant 30 minutes, puis 1,23 g d'acide sulfurique à 98% et 0,92 g d'acide hypophosphoreux à 50% sont introduits dans la dispersion homogène préalablement préparée. Le milieu réactionnel est placé sous un vide partiel de 90 mbars à 45 mbars, et maintenu à une température de 100°C-105°C pendant une durée de 5 heures avec évacuation de l'eau formée au moyen d'un montage de distillation. Le milieu réactionnel est ensuite refroidi à 85°C-90°C et neutralisé par ajout de 1,6 g de soude à 40 %, pour amener le pH d'une solution à 5 % de ce mélange à une valeur d'environ 6,5. La composition ainsi obtenue est ensuite vidangée à une température de 70°C et maintenue à température ambiante de façon à obtenir un solide homogène (composition $W_1$).

La composition ($W_1$) obtenue à l'étape précédente sous la forme d'un solide homogène est ensuite broyée par le moyen d'un dispositif de cryobroyage de marque Micronis , équipé d'un refroidissement à l'azote liquide et d'un broyeur à broche bi rotor fonctionnant à une fréquence de 60 Hz, de façon à obtenir une composition (W) se présentant sous la forme d'une poudre fine dont les caractéristiques analytiques sont rassemblées dans le tableau 1 ci-dessous.

**Exemple comparatif 1.5 : préparation d'une composition solide (X$_1$) constituée d'alcool arachidylique, d'alcool béhénique, d'arachidyl polyglucosides et de béhényl polyglucoside.**

Le procédé de préparation décrit dans l'exemple 1.1 est mis en oeuvre jusqu'à l'obtention de la composition (X$_1$) se présentant sous la forme d'un solide non pulvérulent ; l'étape de broyage n'est pas réalisée.. Les caractéristiques analytiques de la composition (X$_1$) sont indiquées dans le tableau 1 ci-dessous.

**Exemple comparatif 1.6 : préparation d'une composition solide (Y$_1$) constituée d'alcool arachidylique, d'alcool béhénique, d'arachidyl polyglucosides et de béhényl polyglucoside.**

Le procédé de préparation décrit dans l'exemple 1.2 est mis en oeuvre jusqu'à l'obtention de la composition (Y$_1$) se présentant sous la forme d'un solide non pulvérulent ; l'étape de broyage n'est pas réalisée. Les caractéristiques analytiques de la composition (Y$_1$) sont indiquées dans le tableau 1 ci-dessous.

**Exemple comparatif 1.7** : préparation d'une composition solide (W$_1$) constituée d'alcool laurique, d'alcool myristique, cétylique, d'alcool stéarylique, d'alcool arachidylique, d'alcool béhénique, de lauryl polyglucosides, de myristyl polyglucosides, de cetyl polyglucosides, de stéaryl polyglucoside, d'arachidyl polyglucoside et de béhényl polyglucosides.

Le procédé de préparation décrit dans l'exemple 1.4 est mis en oeuvre jusqu'à l'obtention de la composition (W$_1$) se présentant sous la forme d'un solide non pulvérulent ; l'étape de broyage n'est pas réalisée. Les caractéristiques analytiques de la composition (W$_1$) sont indiquées dans le tableau 1 ci-dessous.

**Tableau 1 -** Caractéristiques analytiques des compositions (X), (Y), (X$_1$), (Y$_2$), (Y$_1$), (W) et (W$_1$).

| | (X) | (X$_1$) | (Y) | (Y$_2$) | (Y$_1$) | (W) | (W$_1$) |
|---|---|---|---|---|---|---|---|
| Aspect à 20°C (Détermination visuelle) | Poudre blanche | Solide blanc | Poudre blanche | Poudre blanche | Solide blanc | Poudre blanche | Solide blanc |
| Indice d'acide (NFT 60204) en mg de KOH/g | 0,3 | 0,3 | 0,25 | 0.24 | 0,25 | 0,27 | 0,27 |
| Indice d'hydroxyle (USP XXI NF XVI 01/01/1995) en mg de KOH/g | 214 | 214 | 273 | 298 | 273 | 245 | 245 |
| pH à 5% dans l'eau | 7,1 | 7,1 | 6,1 | 6,1 | 6,1 | 6,5 | 6,5 |
| Eau (% massique) (Norme NFT 73201) | 0,2 | 0,2 | 0,1 | 0,2 | 0,1 | 0,1 | 0,1 |
| Granulométrie Dv (90) en micromètre [2] | 29 | N.A.[1] | 64 | 100 | N.A.[1] | 118 | N.A.[1] |
| Alcools résiduels (CPG) en % massique | 84,5 | 84,5 | 81,0 | 50,8 | 81,0 | 80,1 | 80,1 |
| CPG : (Chromatographie en phase gazeuse) [1]: N.A. = Non applicable [2] La caractéristique analytique Dv(90), qui indique la valeur du diamètre moyen à laquelle 90% du volume des particules de la composition émulsionnante pulvérulente est inférieure, est mesurée à l'aide d'un granulomètre laser MALVERN Mastersizer 2000, équipé d'un dispositif de dispersion de type MS1-Small Volume Sample Dispersion; le diluant utilisé dans cette mesure étant le myristate d'isopropyle. | | | | | | | |

**Principe de la mesure de la caractéristique technique granulométrique Dv(90) par le granulomètre laser MALVERN Mastersizer 2000, équipé du dispositif de dispersion de type MS1-Small Volume Sample Dispersion.**

Le granulomètre laser MALVERN Mastersizer 2000 utilise la diffraction d'un faisceau laser à une longueur d'onde de 632 nanomètres pour mesurer la taille des particules de la composition pulvérulente analysée. De façon générale, les petites particules, présentes dans la composition pulvérulente à analyser, diffractent la lumière du faisceau laser avec une faible intensité mais selon un angle important ; les grosses particules, présentes dans la composition pulvérulente à analyser, diffractent la lumière du faisceau laser avec une forte intensité mais selon un angle faible (selon la théorie de Mie dans Gustav Mie, « Beiträge zur Optik trüber Medien, speziell kolloidaler Metallösungen ». Ann. Phys. Leipzig 25, 377-445 (1908) ; et selon l'approximation de Franhaufer dans Hecht, E., pages 396 et 397, (1987). Optics, 2nd edition. Addison Wesley. ISBN 0-201-11611-1).Le granulomètre laser MALVERN Mastersizer 2000 collecte l'intensité de diffraction de la lumière en fonction de l'angle et relie cette information, par l'intermédiaire de son logiciel, à la taille des particules analysées et présentes dans la composition pulvérulente.

**Mode opératoire de la mesure de la caractéristique technique granulométrique Dv(90) par le granulomètre laser MALVERN Mastersizer 2000, équipé du dispositif de dispersion de type MS1-Small Volume Sample Dispersion.**

○ Une dispersion, comprenant une teneur volumique de 0,0012 % de composition pulvérulente à analyser dans le myristate d'isopropyle, est préparée par mélange du volume nécessaire de la composition pulvérulente à analyser dans le myristate d'isopropyle dans un bécher équipé d'une agitation magnétique. La dispersion ainsi préparée est alors introduite dans le dispositif de dispersion de type MS1-Small Volume Sample Dispersion, équipé d'une pompe dont la vitesse de rotation est réglée à 1500 tours/minute, de façon à faire circuler cette dispersion de la composition pulvérulente à analyser dans le système de mesure du granulomètre laser MALVERN Mastersizer 2000.

○ L'opérateur saisi ensuite sur l'interface du logiciel de mesure du granulomètre laser MALVERN Mastersizer 2000 la valeur de l'indice de réfraction du myristate d'isopropyle, préalablement mesuré à la longueur d'onde de la raie D du Sodium (589,3 nanomètres) et à 20°C, par le moyen d'un réfractomètre RE 40 commercialisé par la société METTLER TOLEDO. Lors de la mesure de la caractéristique technique Dv(90) pour les compositions émulsionnantes pulvérulentes (X), (Y), $(Y_2)$ et (W) selon l'invention, l'indice de réfraction du myristate d'isopropyle, utilisé comme diluant pour les besoins des mesures granulométriques, déterminé à la longueur d'onde de la raie D du Sodium (589,3 nanomètres) et à 20°C par le réfractomètre RE 40 commercialisé par la société METTLER TOLEDO,a été mesuré à une valeur de 1,431.

○ A l'issue de la mesure, effectuée selon le modèle de l'approximation de Franhaufer, le logiciel du granulomètre laser MALVERN Mastersizer 2000 fournit plusieurs caractéristiques analytiques de la composition pulvérulente à analyser dont la caractéristique analytique Dv(90).

**Exemple 2: préparation d'émulsions huile-dans-eau selon un procédé mettant en oeuvre une étape de mélange de la composition émulsionnante pulvérulente selon l'invention dans une phase huileuse.**

On prépare une série d'émulsions huile-dans-eau (Emulsions E1 à E7), dont les compositions sont indiquées dans les tableaux 2 à 4 ci-dessous, en mettant en oeuvre dans le procédé suivant :

**Etape a)** : La composition émulsionnante à tester est ajoutée sur une huile préalablement introduite dans un réacteur porté à une température T1, et le mélange obtenu est homogénéisé pendant 30 minutes par l'intermédiaire d'un agitateur muni d'un mobile de type ancre, à une vitesse de 80 tours/minute.

**Etape b)** : Le polymère polyélectrolyte est introduit sur le mélange préparé à l'issue de l'étape a) maintenu à une température T1. Le mélange résultant est homogénéisé pendant 15 minutes par l'intermédiaire d'un agitateur muni d'un mobile de type ancre, à une vitesse de 80 tours/minute.

**Etape c)** : L'eau est introduite sur le mélange préparé à l'issue de l'étape b), et le mélange résultant est porté à une température T2, puis soumis à une agitation par l'intermédiaire d'un émulseur de type rotor-stator, commercialisé par la société SILVERSON, pendant une durée de 4 minutes à une vitesse de 4000 tours/minute. Le mélange résultant de l'étape c) est ensuite refroidi à température ambiante. La moitié de la quantité de chaque émulsion ainsi préparée est ensuite conservée dans une enceinte climatique isolée et régulée à une température de 20°C pendant 3 mois. L'autre moitié de la quantité de chaque émulsion ainsi préparées est conservée dans une enceinte climatique isolée et régulée à une température de 45°C pendant 3 mois. A l'issue de cette période de 3 mois, l'aspect de chaque émulsion préparée est observé.

Les compositions émulsionnante pulvérulentes (X), (Y), $(Y_2)$ et (W) selon l'invention et les compositions émulsionnantes solides $(X_1)$, $(Y_1)$ et (W1) préparées dans les exemples comparatifs 1.5, 1.6 et 1.7 ont été testées selon le procédé décrit ci-dessus, à pour des températures T1 de préparation de la phase grasse de 25°C, 40°C et 60°C et à des températures T2 d'émulsification de 25°C, 40°C et 60°C.

**Tableau 2 :** Composition et caractérisation des émulsions E1 à E7.

| % massique | Emulsion | | | | | | |
|---|---|---|---|---|---|---|---|
| Emulsionnant | E1 | E2 | E3 | E4 | E5 | E6 | E7 |
| X | 2% | 0% | 0% | 0% | 0% | 0% | 0% |
| $X_1$ | 0% | 2% | 0% | 0% | 0% | 0% | 0% |
| Y | 0% | 0% | 2% | 0% | 0% | 0% | 0% |
| $Y_2$ | 0% | 0% | 0% | 2% | 0% | 0% | 0% |
| $Y_1$ | 0% | 0% | 0% | 0% | 2% | 0% | 0% |
| w | 0% | 0% | 0% | 0% | 0% | 2% | 0% |
| $W_1$ | 0% | 0% | 0% | 0% | 0% | 0% | 2% |
| Huile : Triglycéride C8-C10 | 20% | 20% | 20% | 20% | 20% | 20% | 20% |
| SIMULGEL 600 (4) | 0,5% | 0,5% | 0,5% | 0,5% | 0,5% | 0,5% | 0,5% |
| Eau | Qsp 100% | Qsp 100% | Qsp 100% | Qsp 100% | Qsp 100% | Qsp 100% | Qsp 100% |
| - Sepicide™ HB (3) | 1% | 1% | 1% | 1% | 1% | 1% | 1% |
| T1 (a) | 60°C | 60°C | 60°C | 60°C | 60°C | 60°C | 60°C |
| T2 (b) | 60°C | 60°C | 60°C | 60°C | 60°C | 60°C | 60°C |
| Aspect après 3 mois à 20°C | H (c) | Nf(d) | H | H | Nf | Nf | Nf |
| Aspect après 3 mois à 45°C | H | Nf | H | H | Nf | H | Nf |

(3) SEPICIDE™ HB, qui est un mélange de phénoxyéthanol, de méthylparaben, d'éthylparaben, de propylparaben et de butylparaben, est un agent conservateur commercialisé par la société SEPPIC ;

(4) SIMULGEL™ 600 (acrylamide/sodium acryloyldiméthyl taurate copolymer ; isohexadécane ; polysorbate 80), est une composition épaississante et gélifiante, commercialisée par la société SEPPIC ;

(a) : T1 est la température de préparation du mélange émulsionnant + huile ;

(b) : T2 est la température de mélange de l'émulsion ;

(c) : H : aspect homogène (d) : Nf : émulsion non formée

**Tableau 3 :** Composition et caractérisation des émulsions E8 à E14.

| % massique | Emulsions | | | | | | |
|---|---|---|---|---|---|---|---|
| Emulsionnant | E8 | E9 | E10 | E11 | E12 | E13 | E14 |
| X | 2% | 0% | 0% | 0% | 0% | 0% | 0% |
| $X_1$ | 0% | 2% | 0% | 0% | 0% | 0% | 0% |
| Y | 0% | 0% | 2% | 0% | 0% | 0% | 0% |
| $Y_2$ | 0% | 0% | 0% | 2% | 0% | 0% | 0% |
| $Y_1$ | 0% | 0% | 0%v | 0% | 2% | 0% | 0% |
| W | 0% | 0% | 0% | 0% | 0% | 2% | 0% |
| $W_1$ | 0% | 0% | 0% | 0% | 0% | 0% | 2% |
| Huile : Triglycéride C8-C10 | 20% | 20% | 20% | 20% | 20% | 20% | 20% |
| SIMULGEL 600 | 0,5% | 0,5% | 0,5% | 0,5% | 0,5% | 0,5% | 0,5% |
| Eau | Qsp 100% | Qsp 100% | Qsp 100% | Qsp 100% | Qsp 100% | Qsp 100% | Qsp 100% |
| - Sepicide™ HB | 1% | 1% | 1% | 1% | 1% | 1% | 1% |

(suite)

| % massique | Emulsions | | | | | | |
|---|---|---|---|---|---|---|---|
| Emulsionnant | E8 | E9 | E10 | E11 | E12 | E13 | E14 |
| T1 (a) | 40°C | 40°C | 40°C | 40°C | 40°C | 40°C | 40°C |
| T2 (b) | 40°C | 40°C | 40°C | 40°C | 40°C | 40°C | 40°C |
| Aspect après 3 mois à 20°C | H (c) | Nf (d) | H | H | Nf | H | Nf |
| Aspect après 3 mois à 45°C | H | Nf | H | H | Nf | H | Nf |
| (a) : T1 est la température de préparation du mélange émulsionnant + huile ;<br>(b) : T2 est la température de mélange de l'émulsion ;<br>(c) : H : aspect homogène<br>(d) : Nf : émulsion non formée | | | | | | | |

**Tableau 4 :** Composition et caractérisation des émulsions E15 à E21.

| % massique | Emulsions | | | | | | |
|---|---|---|---|---|---|---|---|
| Emulsionnant | E15 | E16 | E17 | E18 | E19 | E20 | E21 |
| X | 2% | 0% | 0% | 0% | 0% | 0% | 0% |
| $X_1$ | 0% | 2% | 0% | 0% | 0% | 0% | 0% |
| Y | 0% | 0% | 2% | 0% | 0% | 0% | 0% |
| $Y_2$ | 0% | 0% | 0% | 2% | 0% | 0% | 0% |
| $Y_1$ | 0% | 0% | 0%v | 0% | 2% | 0% | 0% |
| W | 0% | 0% | 0% | 0% | 0% | 2% | 0% |
| $W_1$ | 0% | 0% | 0% | 0% | 0% | 0% | 2% |
| Huile : Triglycéride C8-C10 | 20% | 20% | 20% | 20% | 20% | 20% | 20% |
| SIMULGEL 600 | 0,5% | 0,5% | 0,5% | 0,5% | 0,5% | 0,5% | 0,5% |
| <u>Eau</u> | Qsp 100% | Qsp 100% | Qsp 100% | Qsp 100% | Qsp 100% | Qsp 100% | Qsp 100% |
| - Sepicide™ HB | 1% | 1% | 1% | 1% | 1% | 1% | 1% |
| T1 (a) | 25°C | 25°C | 25°C | 25°C | 25°C | 25°C | 25°C |
| T2 (b) | 25°C | 25°C | 25°C | 25°C | 25°C | 25°C | 25°C |
| Aspect après 3 mois à 20°C | H (c) | Nf (d) | H | H | Nf | H | Nf |
| Aspect après 3 mois à 45°C | H | Nf | H | H | Nf | H | Nf |
| (a) : T1 est la température de préparation du mélange émulsionnant + huile ;<br>(b) : T2 est la température de mélange de l'émulsion ;<br>(c) : H : aspect homogène<br>(d) : Nf : émulsion non formée | | | | | | | |

Les résultats compris dans les tableaux 2 à 4 font apparaître que l'utilisation des compositions émulsionnantes pulvérulentes (X), (Y), ($Y_2$) et (W) selon l'invention, permettent d'obtenir une émulsion huile-dans-eau stable au stockage à 20°C et à 45°C, en mettant en oeuvre un procédé de préparation selon l'invention se caractérisant par une température T1 inférieure à 70°C lors de préparation de la phase grasse, et par une température T2 inférieure à 70°C lors de l'étape d'émulsification.

L'utilisation des compositions émulsionnantes comparatives ($X_1$), ($Y_1$) et ($W_1$) se présentant sous la forme de solides non divisés ne permettent pas d'obtenir d'émulsions huile-dans-eau homogènes en mettant en oeuvre un procédé de préparation selon l'invention se caractérisant par une température T1 inférieure à 70°C lors de préparation de la phase grasse, et par une température T2 inférieure à 70°C lors de l'étape d'émulsification.

**EP 2 398 454 B1**

Ainsi, pour une température T1 de préparation du mélange système émulsionnant + huile fixée à 60°C, et pour une température de mélange de l'émulsion T2 fixée à 60°C :

- la composition émulsionnante selon l'invention (X) permet d'obtenir une émulsion homogène et stable au stockage (E1) alors que la composition émulsionnante comparative (X1) ne permet pas de former une émulsion stable et homogène (E2)
- les compositions émulsionnantes selon l'invention (Y) et (Y2) permettent d'obtenir une émulsion homogène et stable au stockage (respectivement E3 et E4) alors que la composition émulsionnante comparative (Y1) ne permet pas de former une émulsion stable et homogène (E5).
- la composition émulsionnante selon l'invention (W) permet d'obtenir une émulsion homogène et stable au stockage (E6) alors que la composition émulsionnante comparative (W1) ne permet pas de former une émulsion stable et homogène (E8).

L'obtention d'émulsions homogènes et stables au stockage par la mise en oeuvre de la composition (X) selon l'invention est également observée lorsque les températures T1 et T2 sont fixées respectivement à 40°C (E8) et à 25°C (E15), alors que la mise en oeuvre de la composition émulsionnante comparative (X1) dans les mêmes conditions de température T1 et T2 ne permet pas de former une émulsion stable et homogène (E9 et E16).

L'obtention d'émulsions homogènes et stables au stockage par la mise en oeuvre des compositions (Y) et (Y2) selon l'invention est également observée lorsque les températures T1 et T2 sont fixées respectivement à 40°C (respectivement E10 et E11) et à 25°C (respectivement E17 et E18), alors que la mise en oeuvre de la composition émulsionnante comparative (Y1) dans les mêmes conditions de température T1 et T2 ne permet pas de former une émulsion stable et homogène (E12 et E19).

L'obtention d'émulsions homogènes et stables au stockage par la mise en oeuvre de la composition (W) selon l'invention est également observée lorsque les températures T1 et T2 sont fixées respectivement à 40°C (E13) et à 25°C (E20), alors que la mise en oeuvre de la composition émulsionnante comparative (W1) dans les mêmes conditions de température T1 et T2 ne permet pas de former une émulsion stable et homogène (E14 et E21).

**Exemple 3: préparation d'émulsions huile-dans-eau selon un procédé mettant en oeuvre une étape de mélange de la composition émulsionnante pulvérulente selon l'invention dans une phase aqueuse.**

On prépare une série d'émulsions huile-dans-eau, dont les compositions sont indiquées dans le tableau 5, en mettant en oeuvre dans le procédé suivant :

**Etape a)** : La composition émulsionnante à tester est ajoutée sur une phase aqueuse préalablement introduite dans un réacteur porté à une température T3, et le mélange obtenu est homogénéisé pendant 30 minutes par l'intermédiaire d'un agitateur muni d'un mobile de type ancre, à une vitesse de 80 tours/minute.
**Etape b)** En parallèle à l'étape a), le polymère polyélectrolyte est introduit sur une huile préalablement introduite dans un réacteur distinct porté à la même température. Le mélange résultant est homogénéisé pendant 15 minutes par l'intermédiaire d'un agitateur muni d'un mobile de type ancre, à une vitesse de 80 tours/minute.
**Etape c)** La phase aqueuse comprenant la composition émulsionnante à tester et préparée à l'issue de l'étape a) est introduite sur le mélange préparé à l'issue de l'étape b), et le mélange résultant est porté à une température T4, puis soumis à une agitation par l'intermédiaire d'un émulseur de type rotor-stator, commercialisé par la société SILVERSON, pendant une durée de 4 minutes à une vitesse de 4000 tours/minute. Le mélange résultant de l'étape c) est ensuite refroidi à température ambiante. La moitié de la quantité de chaque émulsion ainsi préparée est ensuite conservée dans une enceinte climatique isolée et régulée à une température de 20°C pendant 3 mois. L'autre moitié de la quantité de chaque émulsion ainsi préparées est conservée dans une enceinte climatique isolée et régulée à une température de 45°C pendant 3 mois.

A l'issue de cette période de 3 mois, l'aspect de chaque émulsion préparée est observé.
La composition émulsionnante pulvérulente (X) selon l'invention et la composition émulsionnante solide (X$_1$) préparée dans l'exemple comparatif 1.5, ont été testées selon le procédé décrit ci-dessus, à une température T3 de préparation de la phase grasse de 60°C et à une température T4 d'émulsification de 60°C.

17

**Tableau 5 :** Composition et caractérisation des émulsions E22 à E25

| Emulsionnant | Emulsions | | | |
|---|---|---|---|---|
| | E22 | E23 | E24 | E25 |
| X | 2% | 0% | 2% | 0% |
| $X_1$ | 0% | 2% | 0% | 2% |
| Eau | Qsp 100% | Qsp 100% | Qsp 100% | Qsp 100% |
| Sepicide™ HB[3] | 1% | 1% | 1% | 1% |
| Huile : Triglycéride C8-C10 | 10% | 10% | 20% | 20% |
| SIMULGEL 600[4] | 0,5% | 0,5% | 0,5% | 0,5% |
| T3 (e) | 60°C | 60°C | 60°C | 60°C |
| T4 (f) | 60°C | 60°C | 60°C | 60°C |
| Aspect après 3 mois à 20°C | H | Nf | H | H |
| Aspect après 3 mois à 45°C | H | Nf | H | Nf |
| (c) : H : aspect homogène (d) : Nf : émulsion non formée (e) : T3 est la température de préparation du mélange émulsionnant + phase aqueuse ; (f) : T4 est la température de mélange de l'émulsion ; | | | | |

Les résultats compris dans le tableau 5 font apparaître que l'utilisation de la composition émulsionnante pulvérulente (X) selon l'invention, permettent d'obtenir des émulsions huile-dans-eau, comportant des proportions massiques de 10% et de 20% d'huile, stables et homogènes au stockage à 20°C et à 45°C, en mettant en oeuvre un procédé de préparation selon l'invention se caractérisant par une température T3 de 60°C lors de préparation de la phase aqueuse, et par une température T4 de 60°C lors de l'étape d'émulsification (émulsions E22 et E24).

L'utilisation de la composition émulsionnante comparative ($X_1$) se présentant sous la forme d'un solide non pulvérulent ne permettent pas d'obtenir d'émulsions huile-dans-eau, comportant des proportions massiques de 10% et de 20% d'huile, en mettant en oeuvre un procédé de préparation selon l'invention se caractérisant par une température T3 de 60°C lors de préparation de la phase aqueuse, et par une température T4 de 60°C lors de l'étape d'émulsification (émulsions E23 et E25).

**Exemple 4: préparation d'une émulsion huile-dans-eau autobronzante selon un procédé mettant en oeuvre une étape de mélange de la composition émulsionnante pulvérulente selon l'invention dans une phase aqueuse.**

La dihydroxyacétone est un produit couramment utilisé en cosmétique comme un agent de bronzage et/ou de brunissement artificiel de la peau; appliquée sur cette dernière, il permet d'obtenir un effet de bronzage ou de brunissement d'apparence plus ou moins semblable à celui qui peut résulter d'une exposition prolongée au soleil ou sous une lampe à rayonnement ultraviolet. La dihydroxyacétone présente l'inconvénient de se dégrader lors qu'elle est mise en oeuvre à des températures supérieures à 70°C, ce qui se traduit généralement par un jaunissement non souhaité des compositions qui la contiennent.

4.1. On prépare une série d'émulsions huile-dans-eau comprenant de la dihydroxyacétone et les compositions émulsionnantes à tester, dont les compositions sont indiquées dans le tableau 6, en mettant en oeuvre dans le procédé suivant :

**Etape a)** : La composition émulsionnante à tester est ajoutée sur une huile préalablement introduite dans un réacteur porté à une température T5, et le mélange obtenu est homogénéisé pendant 30 minutes par l'intermédiaire d'un agitateur muni d'un mobile de type ancre, à une vitesse de 50 tours/minute.

**Etape b)** : En parallèle à l'étape a), on prépare une phase aqueuse comprenant la dihydroxyacétone dans un réacteur distinct porté à une température de 25°C. Le mélange résultant est homogénéisé pendant 15 minutes par l'intermédiaire d'un agitateur muni d'un mobile de type ancre, à une vitesse de 50 tours/minute.

**Etape c) :** La phase aqueuse préparée à l'issue de l'étape b) et un polymère polyélectrolyte sont aoutés conjointement sur la phase grasse comprenant la composition émulsionnante à tester et préparée à l'issue de l'étape a) à une température T6, puis le mélange résultant est soumis à une agitation par l'intermédiaire d'un émulseur de type rotor-stator, commercialisé par la société SILVERSON, pendant une durée de 4 minutes à une vitesse de 3000 tours/minute.Le mélange résultant de l'étape c) est ensuite refroidi à température ambiante. Les émulsions préparées sont ensuite conservées dans une enceinte climatique isolée et régulée à une température de 20°C pendant 7 jours. A l'issue de cette période de 7 jours, l'aspect de l'émulsion préparée est observé et l'évolution de leur coloration (∆E) telle que décrite ci-dessous est mesurée à l'iade d'un chromamètre Minolta CR200.

**4.2** Principe de la méthode de mesure de la couleur d'une composition cosmétique

L'évolution de la coloration de compositions comprenant de la dihydroxyacétone est estimée par la comparaison au cours du temps d'une caractéristique numérique ∆E, calculée à partir de la mesure de 3 paramètres constitutifs d'une couleur :

- le paramètre L*, variant entre 0 et 100, qui représente la clarté de la nuance ; plus la valeur est élevée, plus la nuance est claire
- le paramètre a*, qui varie de -60 à +60, et exprime toute la gamme du vert (a* = -60) au rouge (a* = +60)
- le paramètre b*, qui varie de -60 à +60 et exprime toute la gamme du bleu (b* = -60) au jaune (b* = +60)

Les paramètres L*, a* et b*, caractérisant une émulsion huile-dans-eau comprenant la dihydroxyacétone, sont mesurés pour les différentes émulsions huile-dans-eau préparées à une température de 25°C, puis la valeur ∆E est alors calculée comme suit :

$$\Delta E = \sqrt{((\Delta L^*)^2 + (\Delta a^*)^2 + (\Delta b^*)^2)},$$

avec :

- $\Delta L^* = L^*$ à t2 - $L^*$ à t1
- $\Delta a^* = a^*$ à t2 - $a^*$ à t1
- $\Delta b^* = a^*$ à t2 - $b^*$ à t1

**4.3** Résultats expérimentaux

Les compositions de chaque émulsion E20 et E21 sont indiquées dans le tableau n°6 ci-après, ainsi que la caractérisation de leur aspect et de l'évolution de leur coloration (∆E) après un stockage de 7 jours 25°C. Les paramètres L*, a* et b*, constitutifs de la couleur de chaque émulsion, sont mesurés avec un chromamètre de type Minolta CR200 commercialisé par la société MINOLTA, de façon à mesurer la valeur ∆E correspondante.

**Tableau 6-** Composition et caractérisation des émulsions E26 et E27.

|  | Emulsions | |
|---|---|---|
| Emulsionnant | E26 | E27 |
| X | 2% | 0% |
| $X_1$ | 0% | 2% |
| Phase Huile | | |
| C12-C15 alkyl benzoate | 10% | 10% |
| Diméthicone DC 200/350 (5) | 5% | 5% |
| Tocophérol | 0,05% | 0,05% |
| Sepicide™ HB | 1,0% | 1,0% |
| Simulgel™ INS 100 (6) | 1,0% | 1,0% |
| Phase aqueuse | | |
| Glycérol | 3,0% | 3,0% |

(suite)

| Phase aqueuse | | |
|---|---|---|
| propylène glycol | 2,0% | 2,0% |
| - Eau | Qsp 100% | Qsp 100% |
| - Acide citrique | Qs pH 4 | Qs pH 4 |
| Agent autobronzant | | |
| Dihydroxyacétone en solution à 50 % dans l'eau | 5,0% | 5,0% |
| T5 (g) | 40°C | 80°C |
| T6 (h) | | |
| Aspect des émulsions après 7 jours à 25°C | Emulsion Homogène | Emulsion Homogène |
| ΔE après 7 jours à 25°C | 27,0 | 4,2 |

(5) DC 200/350 est un cyclométhicone commercialisé par la société Dow Corning.

(6) SIMULGEL™INS 100 (hydroxyethyl acrylate / sodium acryloyldiméthyl taurate copolymer ; isohexadécane; polysorbate 60), est une composition épaississante et gélifiante, commercialisée par la société SEPPIC.

**(g) : T5 est la température de préparation du mélange système émulsionnant + phase huileuse** **(h) : T6 est la température de mélange de l'émulsion**

Les résultats consignés dans le tableau 6 font apparaître que l'émulsion huile-dans-eau E26 comprenant la dihydroxyacétone, préparée avec la composition (X) selon l'invention, en mettant en oeuvre un procédé de préparation selon l'invention se caractérisant par une température T5 de 40°C lors de l'étape a) de préparation de la phase grasse et par une température T6 de 40°C lors de l'étape c) d'émulsification, engendre une évolution réduite de la coloration après un stockage de 7 jours à une température de 25°C par rapport à l'émulsion comparative E27 préparée avec la composition émulsionnante comparative (X₁), en mettant en oeuvre un procédé de préparation se caractérisant par une température T5 de 80°C lors de l'étape a) de préparation de la phase grasse et par une température T6 de 80°C lors de l'étape c) d'émulsification. Ainsi, la valeur de la caractéristique ΔE après 7 jours de stockage à une température de 25°C est de 27,0 pour l'émulsion comparative E27 et de 4,2 pour l'émulsion E26 mettant en oeuvre la composition émulsionnante pulvérulente (X) selon l'invention et le procédé de préparation selon l'invention se caractérisant par une température T5 de 40°C lors de l'étape a) de préparation de la phase grasse et par une température T6 de 40°C lors de l'étape c) d'émulsification.

**Exemple 5 : préparation d'une émulsion huile-dans-eau comprenant une huile volatile selon un procédé mettant en oeuvre une étape de mélange de la composition émulsionnante pulvérulente selon l'invention dans une phase huileuse.**

On prépare une série d'émulsions huile-dans-eau comprenant une huile volatile siliconée, la cyclométhicone DC245 commercialisée par la société DOW CORNING, et les compositions émulsionnantes à tester, dont les compositions massiques sont indiquées dans le tableau 7 pour une masse totale théorique (Mth) d'émulsion de 1000 grammes, en mettant en oeuvre dans le procédé suivant :

**Etape a) :** La composition émulsionnante à tester est ajoutée sur une huile préalablement introduite dans un bêcher de capacité de 2 litres et porté à une température T7, et le mélange obtenu est homogénéisé pendant 30 minutes par l'intermédiaire d'un agitateur muni d'un mobile de type ancre, à une vitesse de 80 tours/minute.

**Etape b)** : Le polymère polyélectrolyte et l'eau sont introduits de façon concomittante sur le mélange préparé à l'issue de l'étape a) maintenu à une température T7. Le mélange résultant est soumis à une agitation par l'intermédiaire d'un émulseur de type rotor-stator, commercialisé par la société SILVERSON, pendant une durée de 5 minutes à une vitesse de 3000 tours/minute et à une température T8. Le mélange résultant de l'étape b) est ensuite refroidi à température ambiante et pesé pour déterminer la masse M1 d'émulsion préparée.

Le rendement (R) de préparation de l'émulsion, exprimé en pourcentage, est calculé comme suit :

$$\text{Rendement (R)} = (M1 \times 100) / Mth$$

La moitié de la quantité de chaque émulsion ainsi préparée est ensuite conservée dans une enceinte climatique isolée et régulée à une température de 20°C pendant 3 mois. L'autre moitié de la quantité de chaque émulsion ainsi préparée est conservée dans une enceinte climatique isolée et régulée à une température de 45°C pendant 3 mois.
A l'issue de cette période de 3 mois, l'aspect de chaque émulsion préparée est observé.

<u>Tableau 7-</u> Composition et caractérisation des émulsions E28 et E29.

|  | Emulsion E28 | Emulsion E29 |
|---|---|---|
| <u>Système émulsionnant</u> |  |  |
| Composition (W) | 3% | 0% |
| - Composition (W$_1$) | 0% | 3% |
| <u>Huile :</u> Cyclométhicone DC 245[7] | 20% | 20% |
| Simulgel™ INS 100[6] | 0,5% | 0,5% |
| Eau | Qsp 100% | Qsp 100% |
| T7 (i) | 40°C | 80°C |
| T8 (j) | 40°C | 80°C |
| Masse théorique Mth<br>Masse M1 d'émulsion préparée<br>Rendement (R) | 1000 g<br>991 g<br>99,1 % | 1000 g<br>903 g<br>90,3 % |
| Aspect après 3 mois à 20°C | Emulsion Homogène | Emulsion Homogène |
| Aspect après 3 mois à 45°C | Emulsion Homogène | Emulsion Homogène |
| (7) DC 245 est une cyclométhicone commercialisé par la société Dow Corning.<br>(i) : T7 est la température de préparation du mélange système émulsionnant + phase huileuse<br>(j) : T8 est la température de mélange de l'émulsion | | |

Les résultats consignés dans le tableau 7 font apparaître que l'émulsion huile-dans-eau E28 comprenant l'huile volatile DC 245, préparée avec la composition (W) selon l'invention, en mettant en oeuvre un procédé de préparation selon l'invention se caractérisant par une température T7 de 40°C lors de <u>l'étape a)</u> de préparation de la phase grasse et par une température T8 de 40°C lors de <u>l'étape b)</u> d'émulsification, se caractérise par un rendement massique de préparation de l'émulsion de 99,1% alors que l'émulsion comparative E29, préparée avec la composition comparative (W$_1$), en mettant en oeuvre un procédé de préparation se caractérisant par une température T7 de 80°C lors de <u>l'étape a)</u> de préparation de la phase grasse et par une température T8 de 80°C lors de <u>l'étape b</u>) d'émulsification, se caractérise par un rendement massique de préparation de l'émulsion de 90,1%.
La composition (W) selon l'invention permet donc de préparer une émulsion eau-dans-huile comprenant une phase volatile avec un meilleur rendement massique, et en diminuant les pertes de l'huile volatile par évaporation pendant la mise en oeuvre du procédé de préparation de l'émulsion huile-dans-eau.

<u>**Formulations**</u>

Dans les formules suivantes, les pourcentages sont exprimés en pourcentage massique pour 100% de la masse de la formulation.

<u>**Exemple 6** : **Crème de soin**</u>

| | |
|---|---|
| Cyclométhicone : | 10% |
| SIMULGEL™ EG : | 0,8% |

(suite)

| | | |
|---|---|---|
| Composition (Y) : | 2% |
| Alcool stéarylique : | 1% |
| Alcool stéarique : | 0,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : | q.s.p. 100% |

**Exemple 7 : Lait solaire**

<u>FORMULE</u>

| | | | |
|---|---|---|---|
| A | Composition (Y): | 3,0% |
| | Huile de sésame : | 5,0% |
| | PARSOL™ MCX : | 5,0% |
| | Carraghénane $\lambda$ : | 0,10% |
| B | Eau : | q.s.p. 100% |
| C | SIMULGEL™ NS : | 0,80% |
| D | Parfum : | q.s. |
| | Conservateur : | q.s. |

<u>MODE OPERATOIRE</u>

Emulsionner B dans A à 60°C puis ajouter C vers 60°C, puis D vers 30°C et ajuster le pH si nécessaire.

**Exemple 8 : Fond de teint hydratant et matifiant**

<u>FORMULE</u>

| | | | |
|---|---|---|---|
| A | eau : | 20,0% |
| | Butylène glycol : | 4,0% |
| | PEG-400 : | 4,0% |
| | PECOSIL™ PS100 : | 1,0% |
| | Hydroxyde de Sodium : | q.s. pH = 9 |
| | Dioxyde de titane : | 7,0% |
| | Talc : | 2,0% |
| | Oxyde de fer jaune : | 0,8% |
| | Oxyde de fer rouge : | 0,3% |
| | Oxyde de fer noir : | 0,05% |
| B | LANOL™ 99 : | 8% |
| | Caprylic capric triglycéride | 8% |
| | Composition (X): | 5,00% |
| C | eau : | q.s.p. 100% |
| | MICROPEARL™ M305 : | 2,0% |
| | EDTA tétrasodé : | 0,05% |
| D | Cyclométhicone : | 4,0% |

(suite)

|   | | |
|---|---|---|
| | Gomme de Xanthane : | 0,2% |
| | SIMULGEL™ EG : | 2,5% |
| E | SEPICIDE™ HB : | 0,5% |
| | SEPICIDE CI : | 0,3% |
| | Parfum : | 0,2% |

MODE OPERATOIRE

Préparer à 60°C, les mélanges B + D et A + C, puis mélanger et émulsionner l'ensemble.

**Exemple 9 : Lait corporel**

| | |
|---|---|
| Composition (Y): | 3,5% |
| LANOL™ 37T : | 8,0% |
| SOLAGUM™ L : | 0,05% |
| Eau : | q.s.p. 100% |
| Benzophénone-3: | 2,0% |
| Diméthicone 350cPs : | 0,05% |
| SIMULGEL™ NS : | 2,5% |
| Conservateur : | 0,2% |
| Parfum : | 0,4% |

**Exemple 10 : Emulsion démaquillante à l'huile d'amandes douces**

| | |
|---|---|
| Composition (Y2) : | 5% |
| Huile d'amandes douces : | 5% |
| Eau : | q.s.p. 100% |
| SIMULGEL™ INS 100 : | 0,3% |
| Glycérine : | 5% |
| Conservateur : | 0,2% |
| Parfum : | 0,3% |

**Exemple 11 : Crème hydratante pour peaux grasses**

| | |
|---|---|
| Composition (Y): | 5% |
| Cétylstéaryloctanoate : | 8% |
| Palmitate d'octyle : | 2% |
| Eau : | q.s.p. 100% |
| SIMULGEL™ NS : | 2,6% |
| MICROPEARL™ M100 : | 3,0% |
| Mucopolysaccharides : | 5% |
| SEPICIDE™ HB : | 0,8% |
| Parfum : | 0,3% |

**Exemple 12 : Crème aux AHA pour peaux sensibles**

| | |
|---|---|
| Mélange de N-lauryl aminoacides : | 0,1% à 5% |

(suite)

| | |
|---|---|
| Aspartate de magnésium et de potassium : | 0,002% à 0,5% |
| LANOL™ 99 : | 2% |
| Composition (Y): | 5,0% |
| Eau : | q.s.p. 100% |
| SIMULGEL™ NS : | 1,50% |
| Acide gluconique : | 1,50% |
| Triéthanolamine (TEA) : | 0,9% |
| SEPICIDE™ HB : | 0,3% |
| SEPICIDE™ CI : | 0,2% |
| Parfum : | 0,4% |

**Exemple 13 : Lait démaquillant**

| | |
|---|---|
| Composition (X): | 3% |
| PRIMOL™ 352 : | 8,0% |
| Huile d'amandes douces : | 2% |
| Eau : | q.s.p. 100% |
| SIMULGEL™ NS : | 0,8% |
| Conservateur : | 0,2% |

**Exemple 14 : Lait solaire**

| | |
|---|---|
| Composition (Y): | 3,5% |
| LANOL™ 37T : | 10,0% |
| PARSOL™ MCX : | 5,0% |
| EUSOLEX™ 4360 : | 2,0% |
| Eau : | q.s.p. 100% |
| SEPIPLUS™ 400 : | 1,8% |
| Conservateur : | 0,2% |
| Parfum : | 0,4% |

**Exemple 15 : Crème aux AHA**

| | |
|---|---|
| Composition (Y) : | 5,0% |
| DEEPALINE™ PVB: | 1,05% |
| LANOL™ 99: | 10,0% |
| Eau : | q.s.p. 100% |
| Acide gluconique : | 1,5% |
| TEA (triéthanolamine) : | 0,9% |
| SIMULGEL™ NS : | 1,5% |
| Parfum: | 0,4% |
| SEPICIDE™ HB: | 0,2% |
| SEPICIDE™ CI: | 0,4% |

**Exemple 16** : **Emulsion bronzante sans soleil**

| | |
|---|---|
| LANOL™ 99 : | 15% |
| Composition (Y) : | 5,0% |
| PARSOL™ MCX : | 3,0% |
| Eau : | q.s.p. 100% |
| Dihydroxyacétone : | 5,0% |
| Phosphate monosodique : | 0,2% |
| SIMULGEL™ NS : | 2,5% |
| Parfum : | 0,3% |
| SEPICIDE™ HB : | 0,8% |
| Hydroxyde de sodium : | q.s. pH=5. |

**Exemple 17** : **Crème de soin**

| | |
|---|---|
| Cyclométhicone : | 10% |
| SIMULGEL™ EG: | 2,8% |
| Composition (Y) : | 4,5% |
| Conservateur : | 0,65% |
| Lysine : | 0,025% |
| EDTA (sel disodique) : | 0,05% |
| Gomme de xanthane : | 0,2% |
| Glycérine : | 3% |
| Eau : | qsp. 100% |

**Exemple 18 : Crème de soin**

| | |
|---|---|
| Cyclométhicone: | 10% |
| SEPIGEL™ 305 : | 0,8% |
| Composition (Y): | 4,5% |
| Perfluoropolyméthylisopropyléther : | 0,5% |
| Conservateur : | 0,65% |
| Lysine : | 0,025% |
| EDTA (sel disodique) : | 0,05% |
| PEMULEN™ TR1 : | 0,2% |
| Glycérine : | 3% |
| Eau: | qsp. 100% |

**Exemple 19** : **Lait corporel**

FORMULE

| | | |
|---|---|---|
| A | Composition (Y2) : | 3,0% |
| | Triheptonate de glycérol : | 10,0% |
| B | Eau : | q.s.p. 100% |

(suite)

| | | | |
|---|---|---|---|
| C | SIMULGEL™ EG : | | 1,0% |
| D | Parfum : | | q.s. |
| | Conservateur : | | q.s. |

<u>MODE OPERATOIRE</u>

Fondre A à environ 40°C. Emulsionner B dans A à 40°C puis ajouter C vers 40°C, puis D.

**Exemple 20 : Lait corporel**

| | |
|---|---|
| Composition (Y) : | 3,5% |
| LANOL™ 37T : | 8,0% |
| SOLAGUM™ L : | 0,05% |
| Eau : | q.s.p. 100% |
| Benzophénone-3 : | 2,0% |
| Diméthicone 350 cPs : | 0,05% |
| SIMULGEL™ NS : | 2,8% |
| Conservateur : | 0,2% |
| Parfum : | 0,4% |

**Exemple 21 : Crème aux AHA**

| | |
|---|---|
| Composition (Y) : | 5,0% |
| DEEPALINE™ PVB : | 1,05% |
| LANOL™ 99 : | 10,0% |
| Eau : | q.s.p. 100% |
| Acide gluconique : | 1,5% |
| TEA (triéthanolamine) : | 0,9% |
| SIMULGEL™ EG : | 1,5% |
| Parfum : | 0,4% |
| SEPICIDE™ HB : | 0,2% |
| SEPICIDE™ CI : | 0,4% |

**Exemple 22 : Lait démaquillant**

| | |
|---|---|
| SIMULSOL™ 165 : | 4% |
| Composition (Y2) : | 1% |
| Caprylate-caprate triglycéride : | 15% |
| PECOSIL™ DCT : | 1% |
| Eau déminéralisée : | qs |
| CAPIGEL™ 98 : | 0,5% |
| SIMULGEL™ INS 100 : | 1% |
| PROTEOL™ APL : | 2% |
| Hydroxyde de sodium : | qsp pH = 7 |

**Exemple 23 : Crème solaire**

| | |
|---|---|
| SIMULSOL™ 165 : | 3% |
| Composition (Y) : | 2% |
| Benzoate C12-C15 : | 8% |
| PECOSIL™ PS 100 : | 2% |
| Diméthicone : | 2% |
| Cyclométhicone : | 5% |
| Para-méthoxy cinnamate d'octyle : | 6% |
| Benzophénone-3: | 4% |
| Oxyde de Titane : | 8% |
| Gomme xanthane : | 0,2% |
| Butylèneglycol : | 5% |
| Eau déminéralisée : | qsp 100% |
| SIMULGEL™ NS : | 1,5% |
| Conservateur, parfum : | qs |

**Exemple 24 : Crème vitaminée**

| | |
|---|---|
| SIMULSOL™ 165 : | 5% |
| Composition (Y) : | 1% |
| Caprylic/capric triglycérides : | 20% |
| Palmitate de vitamine A : | 0,2% |
| Acétate de vitamine E : | 1% |
| MICROPEARL™ M 305 : | 1,5% |
| SIMULGEL™ 600 : | 2% |
| Eau | qsp 100% |
| Conservateur, parfum | qs |

**Exemple 25 : Gel solaire et autobronzant**

| | |
|---|---|
| Composition (Y) : | 3,0% |
| Triheptanoate de glycéryle : | 10,0% |
| DEEPALINE™ PVB : | 1,05% |
| SIMULGEL™ EG : | 2,2% |
| Eau : | qs 100% |
| Dihydroxyacétone : | 5% |
| Parfum: | 0,1% |
| SEPICIDE™ HB : | 0,3% |
| SEPICIDE™ CI : | 0,1% |
| PARSOL™ MCX : | 4,0% |

**Exemple 26 : Crème autobronzante aux α-hydroxy acides**

| | | |
|---|---|---|
| Composition (Y) : | | 5,0% |

(suite)

| | |
|---|---|
| DEEPALINE™ PVB : | 1,05% |
| Lanol™ 99 : | 10,0% |
| Eau : | qs 100% |
| Acide gluconique : | 1,5% |
| Dihydroxyacétone : | 3% |
| Triéthanolamine : | 0,9% |
| SIMULGEL™ EG : | 1,5% |
| Parfum : | 0,4% |
| SEPICIDE™ HB : | 0,2% |
| SEPICIDE™ CI : | 0,4% |

**Exemple 27** : **Crème autobronzante aux α-hydroxy acides pour peau sensible**

| | |
|---|---|
| Mélange de N-lauroyl aminoacides : | 0,1% à 5% |
| Aspartate de magnésium et de potassium : | 0,002% à 0,5% |
| Composition (X): | 5,0% |
| Lanol™ 99 : | 2,0% |
| Eau : | qs 100% |
| Acide lactique : | 1,5% |
| Dihydroxyacétone : | 3,5% |
| Triéthanolamine : | 0,9% |
| SIMULGEL™ NS : | 1,5% |
| Parfum : | 0,4% |
| SEPICIDE™ HB : | 0,3% |
| SEPICIDE™ CI : | 0,2% |

Les définitions des produits commerciaux utilisés dans les exemples, sont les suivantes :

CAPIGEL™ 98 est un épaississant liquide à base de copolymère acrylate commercialisé pas la société SEPPIC.

LANOL™ 99 est de l'isononanoate d'isononyle commercialisé par la société SEPPIC.

Le MICROPEARL™ M 100 est une poudre ultra fine au toucher très doux et à action matifiante commercialisée par la société MATSUMO.

Le SEPICIDE™ CI, imidazolidine urée, est un agent conservateur commercialisé par la société SEPPIC.

PEMULEN™ TR1 est un polymère acrylique commercialisé par GOODRICH. Le SIMULSOL™ 165 est du stéarate de glycérol auto-émulsionnable commercialisée par la société SEPPIC.

Le SEPICIDE™ HB, qui est un mélange de phénoxyéthanol, de méthylparaben, d'éthylparaben, de propylparaben et de butylparaben, est un agent conservateur commercialisé par la société SEPPIC.

Le PARSOL™ MCX est du para-méthoxy cinnamate d'octyle ; commercialisé par la société GIVAUDAN.

Le LANOL™ 37T est du triheptanoate de glycérol, commercialisé par la société SEPPIC.

Le SOLAGUM™ L est un carraghénane commercialisé par la société SEPPIC.

L'EUSOLEX™ 4360 est un filtre solaire commercialisé par la société MERCK.

La DEEPALINE™ PVB, est un hydrolysât de protéines de blé acylé commercialisé par la société SEPPIC.

Le PROTEOL™ APL est un tensioactif moussant, commercialisée par la société SEPPIC.

Le MICROPEARL™ M 305 est une poudre hydrodispersible soyeuse à base de copolymer méthylméthacrylate réticulé.

Le SIMULGEL™ EG : Latex inverse auto-inversible de copolymère tel que ceux décrits dans la publication internationale WO 99/36445 (Dénomination INCI : Sodium acrylate/Sodium acryloyldimethyl taurate copolymer et Isohexadecane et Polysorbate 80) commercialisé par la société SEPPIC.

SEPIPLUS™ 400 : Latex inverse auto-inversible de copolymères tel que ceux décrits dans la publication internationale WO 2005/040230 (Dénomination INCI : Polyacrylate-13 & Polyisobutene & Polysorbate 20), commercialisé par la société SEPPIC.

Le SIMULGEL™ NS : Latex inverse auto-inversible de copolymères épaississants (Dénomination INCI : hydroxyéthyl acrylate/Sodium acryloyldimethyl taurate copolymer et Squalane et Polysorbate 60) commercialisé par la société SEPPIC.

Le SIMULGEL™ INS 100: Latex inverse auto-inversible de copolymères épaississants (Dénomination INCI : hydroxyéthyl acrylate/Sodium acryloyldimethyl taurate copolymer et Isohexadecane et Polysorbate 60) commercialisé par la société SEPPIC.

Le SIMULGEL™ 600 : Latex inverse auto-inversible de copolymères épaississants (Dénomination INCI : acrylamide/Sodium acryloyldimethyl taurate copolymer et Isohexadecane et Polysorbate 80) commercialisé par la société SEPPIC.

Le SEPIGEL™ 305 : Latex inverse auto-inversible de copolymères épaississants (Dénomination INCI : polyacrylamide et C13-C14 isoparaffine et Laureth-7) commercialisé par la société SEPPIC.

Le PRIMOL™ 352 est une huile minérale commercialisée par la société EXXON.

Le PECOSIL™DCT est du sodium Dimethicone PEG-7 Acetyl Methyltaurate commercialisé par la société PHOENIX.

Le PECOSIL™PS 100 est du Dimethicone PEG-7 commercialisé par la société PHOENIX.

**Revendications**

1. Composition pulvérulente C1 comprenant pour 100% de sa masse :

- de 5% massique à 70% massique, plus particulièrement de 10% massique à 50% massique d'au moins un composé de formule (I) :

$$R\text{-}O\text{-}(G)_x\text{-}H \qquad (I)$$

dans laquelle le radical R représente un radical aliphatique linéaire et saturé comportant de 12 à 22 atomes de carbone, G représente le reste d'un sucre réducteur choisi parmi le groupe constitué du glucose, du xylose et de l'arabinose, et x représente un nombre décimal supérieur ou égal à 1 et inférieur ou égal à 10 ;

- de 95% massique à 30% massique, plus particulièrement de 90% massique à 50% massique d'un ou plusieurs alcools de formule (II) :

$$R'\text{-}OH \qquad (II)$$

dans laquelle le radical R', identique ou différent du radical R tel que défini ci-dessus, représente un radical aliphatique linéaire saturé comportant de 12 à 22 atomes de carbone, et dans laquelle au moins 90% en volume de particules sont de diamètre inférieur ou égal à 250 micromètres, et plus particulièrement de diamètre inférieur ou égal à 150 micromètres.

2. Composition pulvérulente C1 telle que définie à la revendication 1, comprenant une proportion massique non nulle d'au moins un composé de formule (Ia) , correspondant à la formule (I) telle que définie précédemment dans laquelle R représente un radical aliphatique linéaire saturé comportant de 20 à 22 atomes de carbone, et une proportion massique non nulle d'au moins un alcool de formule (IIa), correspondant à la formule (II) telle que définie précédemment dans laquelle R' représente un radical aliphatique linéaire saturé comportant de 20 à 22 atomes de carbone.

3. Composition pulvérulente C1 telle que définie à la revendication 2 comprenant pour 100% de sa masse :

- de 5% massique à 20% massique d'au moins un composé de formule (Ia) ;

- de 1,5% massique à 10% massique d'au moins un composé de formule (Ib) correspondant à la formule (I) telle que définie précédemment, dans laquelle R représente un radical aliphatique linéaire saturé comportant de 12 à 14 atomes de carbone ;

- de 1% massique à 10% massique d'au moins un composé de formule (Ic) correspondant à la formule (I) telle que définie précédemment, dans laquelle R représente un radical aliphatique linéaire saturé comportant de 16 à 18 atomes de carbone ;

- de 45% massique à 80% massique d'au moins un composé de formule (IIa) ;

- de 5% massique à 10% massique d'au moins un composé de formule (IIb) correspondant à la formule (II) telle que définie précédemment dans laquelle R' représente un radical aliphatique linéaire saturé comportant de 12 à 14 atomes de carbone ; et

- de 0% massique à 10% massique d'au moins un composé de formule (IIc) correspondant à la formule (II) telle que définie précédemment dans laquelle R' représente un radical aliphatique linéaire saturé comportant de 16 à 18 atomes de carbone.

**4.** Composition pulvérulente C1 telle que définie à la revendication 1 comprenant pour 100% de sa masse :

- de 5% massique à 70% massique d'au moins un composé de formule (Ic), et
- de 95% massique à 30% massique d'au moins un composé de formule (IIc).

**5.** Composition pulvérulente C1, telle que définie à l'une des revendications 1 à 4, dans laquelle au moins 90% en volume de particules sont de diamètre inférieur ou égal à 100 micromètres, et plus particulièrement de diamètre inférieur ou égal à 50 micromètres.

**6.** Utilisation d'une composition pulvérulente C1 telle que définie à l'une des revendications 1 à 5, comme agent émulsionnant pour préparer des émulsions.

**7.** Procédé de préparation d'une émulsion cosmétique huile-dans-eau par émulsification d'une phase grasse P1 avec une phase aqueuse P2, comprenant au moins une étape a) de préparation de ladite phase grasse P1 comprenant le mélange d'au moins une ou de plusieurs huiles et/ou d'une ou de plusieurs cires, avec une quantité efficace de la composition pulvérulente C1, telle que définie à l'une des revendications 1 à 5.

**8.** Procédé tel que défini à la revendication 7, comprenant en outre :

- au moins une étape b) de mélange de la phase grasse P1 obtenue à l'issue de l'étape a), avec une quantité efficace d'un polymère polyélectrolyte pour obtenir une phase P'1, puis
- au moins une étape c) d'émulsification de ladite phase P'1 obtenue à l'issue de l'étape b) avec ladite aqueuse P2.

**9.** Variante du procédé tel que défini à la revendication 8, comprenant au moins une étape b', d'émulsification de ladite phase grasse P1 obtenue à l'étape a) avec ledit polymère polyélectrolyte et ladite phase aqueuse P2.

**10.** Procédé de préparation d'une émulsion cosmétique huile-dans-eau par émulsification d'une phase aqueuse D1 avec une phase grasse D2 comprenant une étape a1) de préparation de ladite phase aqueuse D1 comprenant le mélange d'une quantité efficace de la composition pulvérulente C1, telle que définie à l'une des revendications 1 à 5, avec de l'eau ou une dispersion aqueuse d'un ou de plusieurs ingrédients hydrophiles cosmétiquement acceptables.

**11.** Procédé tel que défini à la revendication 10, comprenant en outre :

- une étape b1) de préparation de ladite phase grasse D2 comprenant le mélange d'au moins une ou de plusieurs huiles et/ou d'une ou de plusieurs cires, avec une quantité efficace d'un un polymère polyélectrolyte ;
- une étape c1) de mélange de ladite phase grasse D2 obtenue à l'issue de l'étape b1) avec ladite phase aqueuse D1 obtenue à l'issue de l'étape a1).

## Patentansprüche

**1.** Pulverzusammensetzung C1, die pro 100 % ihrer Masse Folgendes enthält:

- 5 Massen-% bis 70 Massen-%, insbesondere 10 Massen-% bis 50 Massen-% mindestens einer Verbindung der Formel (I):

$$R-O-(G)_x-H \qquad (I)$$

worin das Radikal R ein lineares und gesättigtes aliphatisches Radikal mit 12 bis 22 Kohlenstoffatomen darstellt, G den Rest eines reduzierenden Zuckers darstellt, der aus der Gruppe bestehend aus Glukose, Xylose und Arabinose ausgewählt ist, und x eine Dezimalzahl darstellt, die größer oder gleich 1 und kleiner oder gleich 10 ist;
- 95 Massen-% bis 30 Massen-%, insbesondere 90 Massen-% bis 50 Massen-% eines oder mehrerer Alkohole der Formel (II):

R'-OH        (II)

worin das Radikal R', das mit dem oben definierten Radikal identisch oder von ihm verschieden ist, ein lineares gesättigtes aliphatisches Radikal mit 12 bis 22 Kohlenstoffatomen darstellt und worin mindestens 90 Volumen-% der Partikel einen Durchmesser haben, der kleiner oder gleich 250 Mikrometer ist, und insbesondere einen Durchmesser, der kleiner oder gleich 150 Mikrometer ist.

**2.** Pulverzusammensetzung (C) nach Anspruch 1, enthaltend ein Massenverhältnis ungleich null mindestens einer Verbindung der Formel (Ia), die der oben definierten Formel (I) entspricht, worin R ein lineares gesättigtes aliphatisches Radikal mit 20 bis 22 Kohlenstoffatomen darstellt, und ein Massenverhältnis ungleich null mindestens eines Alkohols der Formel (IIa), die der oben definierten Formel (II) entspricht, worin R' ein lineares gesättigtes aliphatisches Radikal mit 20 bis 22 Kohlenstoffatomen darstellt.

**3.** Pulverzusammensetzung C1 nach Anspruch 2, die pro 100 % ihrer Masse Folgendes enthält:

- 5 Massen-% bis 20 Massen-% mindestens einer Verbindung der Formel (Ia);
- 1,5 Massen-% bis 10 Massen-% mindestens einer Verbindung der Formel (Ib), die der oben definierten Formel (I) entspricht, worin R ein lineares gesättigtes aliphatisches Radikal mit 12 bis 14 Kohlenstoffatomen darstellt;
- 1 Massen-% bis 10 Massen-% mindestens einer Verbindung der Formel (Ic), die der oben definierten Formel (I) entspricht, worin R ein lineares gesättigtes aliphatisches Radikal mit 16 bis 18 Kohlenstoffatomen darstellt;
- 45 Massen-% bis 80 Massen-% mindestens einer Verbindung der Formel (IIa);
- 5 Massen-% bis 10 Massen-% mindestens einer Verbindung der Formel (IIb), die der oben definierten Formel (II) entspricht, worin R' ein lineares gesättigtes aliphatisches Radikal mit 12 bis 14 Kohlenstoffatomen darstellt; und
- 0 Massen-% bis 10 Massen-% mindestens einer Verbindung der Formel (IIc), die der oben definierten Formel (II) entspricht, worin R' ein lineares gesättigtes aliphatisches Radikal mit 16 bis 18 Kohlenstoffatomen darstellt.

**4.** Pulverzusammensetzung C1 nach Anspruch 1, die pro 100 % ihrer Masse Folgendes enthält:

- 5 Massen-% bis 70 Massen-% mindestens einer Verbindung der Formel (Ic) und
- 95 Massen-% bis 30 Massen-% mindestens einer Verbindung der Formel (IIc).

**5.** Pulverzusammensetzung C1 nach einem der Ansprüche 1 bis 4, in der mindestens 90 Volumen-% der Partikel einen Durchmesser haben, der kleiner oder gleich 100 Mikrometer ist, und insbesondere einen Durchmesser, der kleiner oder gleich 50 Mikrometer ist.

**6.** Verwendung einer Pulverzusammensetzung C1 nach einem der Ansprüche 1 bis 5 als Emulgator zur Herstellung von Emulsionen.

**7.** Verfahren zur Herstellung einer kosmetischen Öl-in-Wasser-Emulsion durch Emulgieren einer Fettphase P1 mit einer wässrigen Phase P2, umfassend mindestens einen Schritt a) des Herstellens der Fettphase P1, der das Mischen mindestens eines oder mehrerer Öle und/oder eines oder mehrerer Wachse mit einer wirksamen Menge der Pulverzusammensetzung C1 nach einem der Ansprüche 1 bis 5 umfasst.

**8.** Verfahren nach Anspruch 7, umfassend ferner:

- mindestens einen Schritt b) des Mischens der am Ende von Schritt a) erhaltenen Fettphase P1 mit einer wirksamen Menge eines Polyelektrolyt-Polymers zur Herstellung einer Phase P'1 und anschließend
- mindestens einen Schritt c) des Emulgierens der am Ende von Schritt b) erhaltenen Phase P'1 mit der wässrigen Phase P2.

**9.** Variante des Verfahrens nach Anspruch 8, umfassend mindestens einen Schritt b') des Emulgierens der am Ende von Schritt a) erhaltenen Fettphase P1 mit dem Polyelektrolyt-Polymer und der wässrigen Phase P2.

**10.** Verfahren zur Herstellung einer kosmetischen Öl-in-Wasser-Emulsion durch Emulgieren einer wässrigen Phase D1 mit einer Fettphase D2, umfassend einen Schritt a1) des Herstellens der wässrigen Phase D1, der das Mischen einer wirksamen Menge der Pulverzusammensetzung C1 nach einem der Ansprüche 1 bis 5 mit Wasser oder einer wässrigen Dispersion eines oder mehrerer kosmetisch unbedenklicher hydrophiler Inhaltsstoffe umfasst.

**11.** Verfahren nach Anspruch 10, umfassend ferner:

- einen <u>Schritt b1</u>) des Herstellens der Fettphase D2, der das Mischen mindestens eines oder mehrerer Öle und/oder eines oder mehrerer Wachse mit einer wirksamen Menge eines Polyelektrolyt-Polymers umfasst,
- einen <u>Schritt c1</u>) des Mischens der am Ende von <u>Schritt b1</u>) erhaltenen Fettphase D2 mit der am Ende von <u>Schritt a1</u>) erhaltenen wässrigen Phase D1.

**Claims**

**1.** Pulverulent composition C1 comprising, for 100 % of its weight:

- from 5 % by weight to 70 % by weight, more particularly from 10 % by weight to 50 % by weight of at least one compound of formula (I):

$$R-O-(G)_x-H \qquad (I)$$

wherein the radical R represents a saturated and linear aliphatic radical containing from 12 to 22 carbon atoms, G represents the residue of a reducing sugar selected from the group consisting of glucose, xylose and arabinose, and x represents a decimal number greater than or equal to 1 and less than or equal to 10; and
- from 95 % by weight to 30 % by weight, more particularly from 90 % by weight to 50 % by weight of one or more alcohols of formula (II):

$$R'-OH \qquad (II)$$

wherein the radical R', which may be identical to or different from the radical R as defined above, represents a saturated linear aliphatic radical containing from 12 to 22 carbon atoms, and wherein at least 90 % by volume of particles have a diameter of less than or equal to 250 micrometres, and more particularly have a diameter of less than or equal to 150 micrometres.

**2.** Pulverulent composition C1 according to claim 1, comprising a non-zero proportion by weight of at least one compound of formula (Ia), corresponding to formula (I) as defined above, wherein R represents a saturated linear aliphatic radical containing from 20 to 22 carbon atoms, and a non-zero proportion by weight of at least one alcohol of formula (IIa), corresponding to formula (II) as defined above, wherein R' represents a saturated linear aliphatic radical containing from 20 to 22 carbon atoms.

**3.** Pulverulent composition C1 according to claim 2 comprising, for 100 % of its weight:

- from 5 % by weight to 20 % by weight of at least one compound of formula (Ia);
- from 1.5 % by weight to 10 % by weight of at least one compound of formula (Ib) corresponding to formula (I) as defined above, wherein R represents a saturated linear aliphatic radical containing from 12 to 14 carbon atoms;
- from 1 % by weight to 10 % by weight of at least one compound of formula (Ic) corresponding to formula (I) as defined above, wherein R represents a saturated linear aliphatic radical containing from 16 to 18 carbon atoms;
- from 45 % by weight to 80 % by weight of at least one compound of formula (IIa);
- from 5 % by weight to 10 % by weight of at least one compound of formula (IIb) corresponding to formula (II) as defined above, wherein R' represents a saturated linear aliphatic radical containing from 12 to 14 carbon atoms; and
- from 0 % by weight to 10 % by weight of at least one compound of formula (IIc) corresponding to formula (II) as defined above, wherein R' represents a saturated linear aliphatic radical containing from 16 to 18 carbon atoms.

**4.** Pulverulent composition C1 according to claim 1 comprising, for 100 % of its weight:

- from 5 % by weight to 70 % by weight of at least one compound of formula (Ic), and
- from 95 % by weight to 30 % by weight of at least one compound of formula (IIc).

**5.** Pulverulent composition C1 according to any of claims 1 to 4, wherein at least 90 % by volume of the particles have a diameter of less than or equal to 100 micrometres, and more particularly a diameter of less than or equal to 50 micrometres.

6. Use of a pulverulent composition C1 according to any of claims 1 to 5 as an emulsifier for preparing emulsions.

7. Method for preparing an oil-in-water cosmetic emulsion by emulsifying a fatty phase P1 with an aqueous phase P2, comprising at least one step a) of preparing said fatty phase P1 which comprises mixing at least one or more oils and/or one or more waxes with an effective amount of the pulverulent composition C1 according to any of claims 1 to 5.

8. Method according to claim 7, further comprising:

   - at least one step b) of mixing the fatty phase P1 obtained at the end of step a) with an effective amount of a polyelectrolyte polymer in order to obtain a phase P'1, then
   - at least one step c) of emulsifying said phase P'1 obtained at the end of step b) with said aqueous P2.

9. Variant of the method according to claim 8, comprising at least one step b' of emulsifying said fatty phase P1 obtained in step a) with said polyelectrolyte polymer and said aqueous phase P2.

10. Method for preparing an oil-in-water cosmetic emulsion by emulsifying an aqueous phase D1 with a fatty phase D2 comprising a step a1) of preparing said aqueous phase D1 which comprises mixing an effective amount of the pulverulent composition C1 according to any of claims 1 to 5, with water or an aqueous dispersion of one or more cosmetically acceptable hydrophilic ingredients.

11. Method according to claim 10, further comprising:

   - a step b1) of preparing said fatty phase D2 which comprises mixing at least one or more oils and/or one or more waxes with an effective amount of a polyelectrolyte polymer;
   - a step c1) of mixing said fatty phase D2 obtained at the end of step b1), with said aqueous phase D1 obtained at the end of step a1).

## RÉFÉRENCES CITÉES DANS LA DESCRIPTION

**Documents brevets cités dans la description**

- WO 9206778 A
- WO 9513863 A
- WO 9637285 A
- WO 9847610 A
- FR 2784904
- FR 2756195
- EP 0992508 A
- WO 0056438 A
- FR 2830464
- WO 9637286 A
- WO 9936445 A
- WO 2005040230 A

**Littérature non-brevet citée dans la description**

- **P. BOWEN.** Particle Size Distribution Measurement from Millimeters to Nanometers and from Rods to Platelets. *J. Dispersion Science and Technology,* 2002, vol. 23 (5), 631-662
- **MICHEL ; IRENE ASH.** Thesaurus of Chemical Products. Chemical Publishing Co, Inc, 1986, vol. I, 211
- **GUSTAV MIE.** Beiträge zur Optik trüber Medien, speziell kolloidaler Metallösungen. *Ann. Phys. Leipzig,* 1908, vol. 25, 377-445
- **HECHT, E.** Optics. Addison Wesley, 1987, 396-397